# EUROPEAN PATENT APPLICATION

(11) **EP 3 533 619 A1**
(43) Date of publication of application: **04.09.2019**
(21) Application number: 18159670.1
(22) Date of filing: 02.03.2018
(51) Int. Cl.: B41M 5/333, C07C 311/15, C07C 233/64, B41M 5/327, B41M 5/337

(54) **HEAT SENSITIVE RECORDING MATERIAL AND COLOR DEVELOPER**

(71) Applicant: BASF SE, 67056 Ludwigshafen am Rhein (DE)
(72) Inventor: The designation of the inventor has not yet been filed
(74) Representative: BASF IP Association

(57) **Abstract**

The present invention relates to a heat sensitive recording material, a novel compound, a process for its manufactures, and the use of a novel compound as color developer.

## Description

The present invention relates to a heat sensitive recording material comprising a novel color developer, a novel color developer, a process for its manufacture and its use.

For thermographic printing of point of sales receipts so called thermal paper is used which forms a colored image by heating. It is composed of three main components, the color former, the sensitizer and the color developer which forms the colored image by complexation with the color former. The widely used developer bisphenol A is inexpensive and technically sufficient, but under current European chemical legislation this substance is classified as "toxic for reproduction" and suspected of endocrine disruption activity in both humans and in the environment. Bisphenol A will also be restricted in thermal papers placed on the European market from January 2020. Another phenolic color developer, bisphenol S is also under investigation.

Among non-phenolic color developers in commercial use, sulfonyl urea derivatives, described in example in EP 526072 or in particular in WO00/35679 such as N-p-toluenesulfonyl-N'-3-(p-toluenesulfonyloxy)phenylurea, or urea derivatives such as described in EP 2923851, offer an alternative to the widely used phenolic products. However, despite being technically viable alternatives to phenolic color developers, their synthesis requires the use of specialty and semi-specialty raw materials. Consequently, they are not ideal in cost-effectiveness terms for widespread use in Point Of Sales and/or economy grades of thermal paper.

JP 2016-083858 A1 describes a further alternative of a thermosensitive recording material comprising a color developer which, however, is prepared in a three-step procedure.

Accordingly, it is an object of this invention to provide a non-phenolic color developer for use in heat sensitive recording materials that is both a technically suitable and cost-effective alternative to the hitherto used phenolic and non-phenolic color developers.

The present invention is directed to a heat sensitive recording material, comprising
a) a color forming compound, and
b) a color developer of the formula (I) 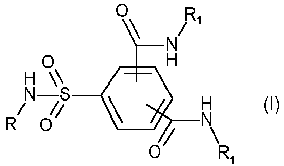 wherein
   R and R₁ are each independently of each other hydrogen, C₁-C₁₈-alkyl, C₁-C₈-alkoxy-C₁-C₈-alkyl, (R₉)₂N-C₁-C₈-alkyl, wherein Rg stands for C₁-C₈-alkyl or C₅-C₆-cycloalkyl, or a radical of formula (II) 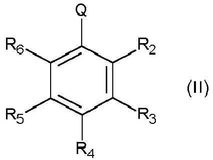 wherein R₂, R₃, R₄, R₅, R₆ are each independently of each other hydrogen, C₁-C₈-alkyl, -NH-C(=O)-R₇, -C(=O)-NH-R₇, wherein R₇ stands for C₁-C₈-alkyl, -C(=O)OR₈, wherein R₈ stands for C₁-C₈-alkyl, halogen such as fluorine, chlorine, bromine, preferably chlorine, or wherein R₂ and R₃, or R₄ and R₅ or both, or wherein R₃ and R₄, or R₅ and R₆ or both, or wherein (R₂ and R₃) as well as (R₅ and R₆), can stand together for a hydrocarbon diradical with 3 or 4 carbon atoms such as trimethylene, tetramethylene, propenylene, 2-butenylen, 1,3-butadienylen,
   and Q stands for a single bond or C₁-C₈-alkylene, which can be branched or unbranched and wherein the main chain may contain one or more oxygen atoms between two carbon atoms in case of more than 2 carbon atoms, preferred for a single bond, -CH₂-, -CH₂-CH₂-, -CH₂-O-, -CH₂-CH₂-O-, -CH₂-CH₂-CH₂-, -CH₂-CH₂-CH₂-CH₂-, -C(Me)H-, -C(Et)H-, -C(n-Pr)H-, -C(i-Pr)H-, -C(n-Bu)H-, -C(i-Bu)H-, -C(sec-Bu)H-, -C(tert-Bu)H-, -C(Me)HCH₂-, -CMe₂CH₂-, most preferred for -CH₂-, -CH₂-CH₂-, -CH₂-CH₂-O- and -C(Me)H-.

C₁-C₁₈-alkyl usually stands for methyl, ethyl, propyl, isopropyl, n-butyl, isobutyl, sec-butyl, tert-butyl, amyl, tert-amyl (1,1-dimethylpropyl), 1,1,3,3-tetramethylbutyl, n-hexyl, 2-methylpentyl, neopentyl, n-heptyl, 2-ethyl-hexyl or n-octyl, n-nonyl, n-decyl, n-undecyl, n-dodecyl, n-tridecyl, n-tetradecyl, n-pentadecyl, n-hexadecyl, n-heptadecyl, n-octadecyl preferably for C₁-C₈-alkyl such as methyl, ethyl, propyl, isopropyl, n-butyl, isobutyl, sec-butyl, tert-butyl, amyl, tert-amyl (1,1-dimethylpropyl), 1,1,3,3-tetramethylbutyl, n-hexyl, 2-methylpentyl, neopentyl, n-heptyl, 2-ethyl-hexyl or n-octyl, as well as for tridecyl, in particular for methyl, ethyl, n-propyl, isopropyl, n-butyl, isobutyl, sec-butyl, tert-butyl, 2-ethyl-hexyl and tridecyl.

C₁-C₈-alkyl usually stands for methyl, ethyl, propyl, isopropyl, n-butyl, isobutyl, sec-butyl, tert-butyl, amyl, tert-amyl (1,1-dimethylpropyl), 1,1,3,3-tetramethylbutyl, n-hexyl, 2-methylpentyl, neopentyl, n-heptyl, 2-ethyl-hexyl or n-octyl, preferably for methyl, ethyl, n-propyl, isopropyl, n-butyl, isobutyl, sec-butyl, tert-butyl.

C₁-C₈-alkoxy usually stands for methoxy, ethoxy, n-propoxy, isopropoxy, n-butyloxy, n-pen-tyloxy, n-hexyloxy, n-heptyloxy, n-octyloxy, preferred for C₁-C₆-alkoxy such as methoxy, ethoxy, n-propoxy, isopropoxy, n-butyloxy, n-hexyloxy.

C₁-C₈-alkoxy-C₁-C₈-alkyl usually stands for methoxymethyl, ethoxymethyl, n-propoxymethyl, isopropoxymethyl, n-butyloxymethyl, n-pentyloxymethyl, n-hexyloxymethyl, n-hepty-loxymethyl, n-octyloxymethyl, methoxyethyl, ethoxyethyl, n-propoxyethyl, isopropoxyethyl, n-butyloxyethyl, n-pentyloxyethyl, n-hexyloxyethyl, n-heptyloxyethyl, n-octyloxyethyl, methoxymethyl, 1-methoxyethyl, 2-methoxyethyl, 3-methoxy-n-propyl, 1-methoxy-2-propyl, 4-methoxy-n-butyl, 5-methoxy-n-pentyl, 6-methoxy-n-hexyl, 7-methoxy-n-heptyl, 8-methoxy-n-octyl, preferred 2-methoxy-ethyl.

C₅-C₆-cycloalkyl usually stands for cyclopentyl or cyclohexyl, preferably for cyclohexyl.

Preferred (R₉)₂N-C₁-C₈-alkyl groups are 2-(dimethylamino)-methyl, 2-(dimethylamino)-ethyl, 2-(diethylamino)-ethyl, 2-(diisopropylamino)-ethyl, 2-(n-propylamino)-ethyl, 3-(dimethyla-mino)-propyl, 3-(cyclohexylamino)-propyl.

In case, wherein R₂ and R₃, or R₄ and R₅ or both, or wherein R₃ and R₄, or R₅ and R₆ or both, or wherein (R₂ and R₃) as well as (R₅ and R₆), can stand together for a hydrocarbon diradical with 3 or 4 carbon atoms such as trimethylene, tetramethylene, propenylene, 2-butenylen, 1,3-butadienylen, preferably R₂ and R₃ together stand for tetramethylene or R₂ and R₃ together stand for tetramethylene.

Preferred radicals of formula (II) are phenyl, benzyl, 3-methylphenyl, 2,6-diethylphenyl, o-isopropylphenyl, p-acetamidophenyl, 1-phenylethyl, 2-phenylethyl, 2-phenoxyethyl, 1-tetralino, 2-tetralino.

Preferred heat sensitive recording materials comprise
a) a color forming compound, and
b) a color developer of the formula (Ia) 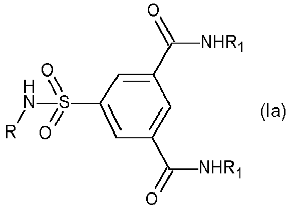 or a color developer of the formula (Ib)

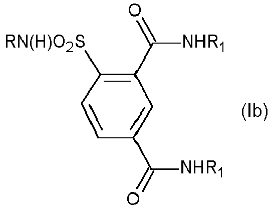

Most preferred is a heat sensitive recording material comprising
a) a color forming compound, and
b) a color developer of the formula (Ia).

A further preferred embodiment of the present invention relates to a heat sensitive recording material comprising
a) a color forming compound,
b) a color developer of the formula (Ia)
   and
c) a bisamide of the formula (Ic) 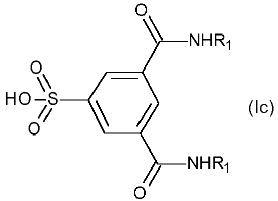 wherein the amount of bisamide (Ic) can be chosen in the range of 0.01 to 10 mol-% related to color developer (Ib).

The invention further relates to a compound of formula I, preferred to compounds (Ia) and (Ib), more preferred to compounds (Ia). Most preferred compounds (Ia) are 5-(N-benzyl-sulfonylamido)-(N',N"-dibenzyl)-isophthalic acid-diamide, 5-(N-3-methylphenyl-sulfonylamido)-(N',N"-bis-(3-methylphenyl)-isophthalic acid-diamide, 5-(N-2,6-diethylphenyl-sulfonylamido)-(N',N"-bis-(2,6-diethylphenyl)-isophthalic acid-diamide, 5-(N-phenyl-sulfonylamido)-(N',N"-bisphenyl)-isophthalic acid-diamide, 5-(N-o-isopropyl-phenyl-sulfonylamido)-(N',N"-bis-(o-isopropylphenyl)-isophthalic acid-diamide, 5-(N-p-acetamido-phenyl-sulfonylamido)-(N',N"-bis-(p-acetamido-phenyl)-isophthalic acid-diamide, 5-(N-1-tetralino-sulfonylamido)-(N',N"-bis-(1-tetralino)-isophthalic acid-diamide, 5-(N-3-methylphenyl-sulfonylamido)-(N',N"-bis-(3-methylphenyl)-isophthalic acid-diamide, 5-(N-1-phenylethyl-sulfonylamido)-(N',N"-bis-(1-phenylethyl)-isophthalic acid-diamide, 5-(N-2-phenylethyl-sulfonylamido)-(N',N"-bis-(2-phenylethyl)-isophthalic acid-diamide, 5-(N-2,6-diethylphenyl-sulfonylamido)-(N',N"-bis-(2,6-diethylphenyl)-isophthalic acid-diamide, 5-(N-n-butyl-sulfonylamido)-(N',N"-di-n-butyl-isophthalic acid-diamide, 5-(N-2-ethylhexyl-sulfonylamido)-(N',N"-di-2-ethylhexyl-isophthalic acid-diamide, 5-(N-benzyl-sulfonylamido)-(N',N"-diphenyl)-isophthalic acid-diamide, 5-(N-phenyl-sulfonylamido)-(N',N"-dibenzyl)-isophthalic acid-diamide, 5-(N-benzyl-sulfonylamido)-(N',N"-bis-(3-methyl-phenyl)-isophthalic acid-diamide, 5-(N-butyl-sulfonyl-amido)-(N',N"-bis-(3-methyl-phenyl)-isophthalic acid-diamide, 5-(N-1-phenyl-ethyl-sulfonyl-amido)-(N',N"-bis-(3-methyl-phenyl)-isophthalic acid-diamide, 5-(N-2-phenyl-ethyl-sulfonyl-amido)-(N',N"-bis-(3-methyl-phenyl)-isophthalic acid-diamide, 5-(N-2-methoxy-ethyl-sul-fonylamido)-(N',N"-bis-(3-methyl-phenyl)-isophthalic acid-diamide, 5-(N-n-octyl-sulfonyl-amido)-(N',N"-bis-(3-methyl-phenyl)-isophthalic acid-diamide, 5-(N-benzyl-sulfonylamido)-(N',N"-bis-(2,6-diethyl-phenyl)-isophthalic acid-diamide, 5-(N-n-octyl-sulfonylamido)-(N',N"-bis-(2,6-diethyl-phenyl)-isophthalic acid-diamide, 5-(N-2-phenoxy-ethyl-sulfonylamido)-(N',N"-bis-(2,6-diethyl-phenyl)-isophthalic acid-diamide.

A further embodiment of the present inventions relates to polymorphic modifications, in particular to 5-(N-3-methylphenyl-sulfonylamido)-(N',N"-bis-(3-methylphenyl)-isophthalic acid-diamide, where three different polymorphic modifications were found: an α-modification having a melting point of 211.2°C (determined using differential scanning colorimetry, DSC), a β-modification having a melting point of 192.2°C (measured via DSC), and a γ-modification having a melting point of 215.6°C (measured via DSC).

Another preferred embodiment of this invention relates to the process for the manufacture of a compound with formula (I), wherein R and R₁ are the same, comprising reacting 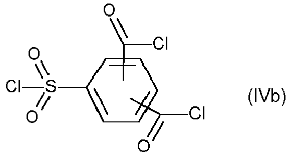 preferably 5-sulfonyl chloride-isophthalic acid dichloride, with an amine RNH₂.

Compounds (IVb) are known in the art and can be prepared according to known methods. In particular, similar methods can be applied as described for the synthesis of 5-sulfonyl chloride-isophthalic acid dichloride below.

Due to its instability against hydrolysis, the acid chloride (IVb), preferably 5-sulfonyl chloride-isophthalic acid dichloride, is produced shortly before its reaction with the above amine RNH₂ or the acid chloride (IVb), preferably 5-sulfonyl chloride-isophthalic acid dichloride, can be used, which was stored under the exclusion of water, for example under an inert atmosphere and possibly also under low temperature conditions.

Usually, a commercially available sulfo-isophthalic compound having the general formula (IVa) 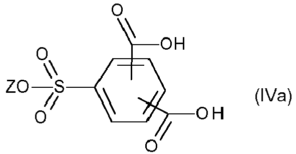 preferably of formula (IV) 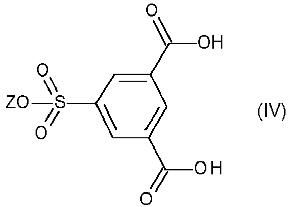 wherein Z stands for hydrogen or an alkali metal such as sodium or potassium, preferably for sodium, is chlorinated with a common chlorination agent.

The acid chloride (IVb), preferably 5-sulfonyl chloride-isophthalic acid dichloride, obtained can be isolated if desired by usual means such as pouring the reaction mixture in an ice-water mixture followed by a separation step such filtration, decantation, centrifugation, extraction etc. The such obtained reaction mixture or the isolated product, preferably the isolated product, can then be used as is in the following reaction or for example freeze-dried or in an inert atmosphere, if it is desired to store 5-sulfonyl chloride-isophthalic acid dichloride without premature hydrolysis.

As chlorination agent, the usual common compounds such as thionyl chloride, POCl₃, PCl₅, or oxalyl chloride can be used.

Depending on the reactivity of the chlorination agent, the amount of chlorination agent relative to the amount of a compound of formula (IV) is chosen as well as the use of a catalyst.

For example, highly reactive chlorination agents are PCl₃, PCl₅ and POCl₃ can be used without any catalysts and in a slight excess such as in a molar ratio of chlorination agent to compound (IV) in the range of from 1:1 to 10:1, preferably from 1.1:1 to 5:1. Less reactive chlorination agents such as thionyl chloride or oxalyl chloride are used in general in a higher excess, for example here a molar ratio of chlorination agent to compound (IV) is chosen in the range of from 1:2 to 20:1, preferably from 5:1 to 20:1, more preferably from 10:1 to 16:1. In a particularly preferred embodiment thionyl chloride is reacted with the sodium salt of 5-sulfo-isophthalic acid in a molar ratio in the range of 5:1 to 20:1, preferably from 10:1 to 16:1.

In another preferred embodiment, a catalyst is used during the chlorination such as dimethylformamide or pyridine. Most preferred is the combination of thionyl chloride and dimethylformamide.

In case a catalyst is used, the molar amount of the catalyst to compound (IV) can be chosen in the molar range of 0.01:1 to 2:1, preferably from 0.1:1 to 0.85:1. In a particularly preferred embodiment, dimethyl formamide is used in a molar ratio in the range of from 0.01:1 to 2:1, preferably from 0.1:1 to 0.85:1, relative to the sodium salt of 5-sulfo-isophthalic acid.

Usually, the chlorination is carried out without a solvent. However, if desired, a solvent can be used such as aprotic aromatic solvents like toluene or a halogenated benzene such as chlorobenzene od 1,2-dichlorobenzene.

In case a solvent is used, the amount can be chosen in a wide range and is not critical based on observations up to now. Usually, the amount depends on the on the solubility of the educts in the chosen solvent. In a preferred embodiment, for example, the weight ratio of the solvent to compound (IV) can be chosen in the range of from 300 to 1, particular from 10 to 1.

Depending on the reactivity of the used chlorination agent and the possible use of a catalyst, the appropriate temperature range is chosen. As a rule, the temperature can be chosen in the range of from 15°C to the boiling point of the reaction mixture, preferably from 15°C to 75°C. In a preferred embodiment, the chlorination with thionyl chloride is carried out under reflux conditions at ambient pressure, i.e. between 70 and 75°C.

Of course, the reaction time usually depends inter alia on the reactivity of the chlorination agent, the chosen temperature and the use of a catalyst, and can be determined easily by a person skilled in the art for example in checking whether the generation of hydro chloride or SO₂ has seized by well-known methods in the art such as using wetted pH-indicator paper. As a rule, the reaction time can be chosen in the range of from 1 h to 10 hours, preferably from 3 to 6 hours.

Although the obtained reaction mixture could be used as is for further processing, it is preferred to remove any unreacted chlorination agent and possible side reaction products first, for example by separating the reaction product with known methods such as decantation, filtration, centrifugation or other work-up methods.

In a preferred embodiment, the reaction mixture obtained after the chlorination step is work-up by the addition of cold solvent such as water, or an organic solvent such as toluene, tetrahydrofuran, wherein cold solvent means the solvent has a temperature in the range of (-5) to 10°C. It goes without saying that also the cold solvent could be added to the reaction mixture. Preferred is the use of water having a temperature between 0 and 10°C or a mixture of ice and water, to the reaction mixture or vice versa. Preferably, any excess chlorination agent such a thionyl chloride can be distilled off prior to that.

Usually, the weight ratio of water or iced water to compound (IV) is chosen in the range of 1:1 to 200:1, preferably from 5:1 to 50:1, more preferably from 15:1 to 25:1.

Preferably the temperature of this reaction mixture is chosen between 0 and 15°C, preferably between 0 and 10°C, and more preferably between 0 and 5°C.

Generally, the duration of this work-up step is not critical and depends as a rule on the excess amount of chlorination agent left in the reaction mixture. Based on observations today, the duration can be chosen in the range of 10 to 180 minutes, preferably from 20 to 120 minutes.

If desired the thus obtained and treated product can be stored at a temperature preferably below 10°C, more preferably between (-30) and 10°C, even more preferred between (-25) and (-10)°C. In a preferred embodiment, the obtained product is "deep-frozen" using a commercially available deep freezer (e.g. Liebherr Comfort GS1663) at a temperature range between (-20) and (-18)°C, optionally followed by a freeze drying step using a freeze-dryer (such as Christ Alpha 2-4 LSC) operating under reduced atmospheric pressure such as in the range of 10 to 0.1 mbar.

Appropriate amines are either known, commercially available or can be synthesized according to known methods such as the reduction of the corresponding nitro compounds.

Preferred amines RNH₂ can be ammonia, C₁-C₁₈-alkylamine, C₁-C₈-alkoxy-C₁-C₈-alkylamine, (R₉)₂N-C₁-C₈-alkylamine, wherein Rg stands for C₁-C₈-alkyl or C₅-C₆-cycloalkyl, or an amine of formula (IIa) 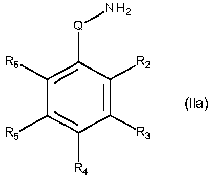 wherein R₂, R₃, R₄, R₅, R₆ are each independently of each other hydrogen, C₁-C₈-alkyl, -NH-C(=O)-R₇, -C(=O)-NH-R₇, wherein R₇ stands for C₁-C₈-alkyl, -C(=O)OR₈, wherein R₈ stands for C₁-C₈-alkyl, halogen such as fluorine, chlorine, bromine, preferably chlorine, or wherein R₂ and R₃, or R₄ and R₅ or both, or wherein R₃ and R₄, or R₅ and R₆ or both, or wherein (R₂ and R₃) as well as (R₅ and R₆), can stand together for a hydrocarbon diradical with 3 or 4 carbon atoms such as trimethylene, tetramethylene, propenylene, 2-butenylen, 1,3-butadienylen, preferably R₂ and R₃ together stand for tetramethylene or R₂ and R₃ together stand for tetramethylene,
and Q stands for a single bond or C₁-C₈-alkylene, which can be branched or unbranched and wherein the main chain may contain one or more oxygen atoms between two carbon atoms in case of more than 2 carbon atoms, preferred for a single bond, -CH₂-, -CH₂-CH₂-, -CH₂-O-, -CH₂-CH₂-O-, -CH₂-CH₂-CH₂-, -CH₂-CH₂-CH₂-CH₂-, -C(Me)H-, -C(Et)H-, -C(n-Pr)H-, -C(i-Pr)H-, -C(n-Bu)H-, -C(i-Bu)H-, -C(sec-Bu)H-, -C(tert-Bu)H-, -C(Me)HCH₂-, -CMe₂CH₂-, most preferred for -CH₂-, -CH₂-CH₂-, -CH₂-CH₂-O- and -C(Me)H-.

C₁-C₁₈-alkylamine can be methylamine, ethylamine, n-propylamine, isopropylamine, n-butylamine, isobutylamine, sec-butylamine, tert-butylamine, n-pentylamine, tert-amylamine (1,1-dimethylpropylamine), 1,1,3,3-tetramethylbutylamine, n-hexylamine, 2- methylpentylamine, neopentylamine, n-heptylamine, 2-ethyl-hexylamine, n-octylamine, n-nonylamine, n-decylamine, n-undecylamine, n-dodecylamine, n-tridecylamine, n-tetradecylamine, n-penta-decylamine, n-hexadecylamine, n-heptadecylamine, n-octadecylamine, preferably for C₁-C₈-alkylamine such as methylamine, ethylamine, n-propylamine, isopropylamine, n-butylamine, isobutylamine, sec-butylamine, tert-butylamine, n-pentylamine, tert-amylamine (1,1-dimethylpropylamine), 1,1,3,3-tetramethylbutylamine, n-hexylamine, 2- methylpentylamine, neopentylamine, n-heptylamine, 2-ethyl-hexylamine, n-octylamine as well as for tridecylamine, in particular for methylamine, ethylamine, n-propylamine, isopropylamine, n-butylamine, isobutylamine, sec-butylamine, tert-butylamine, 2-ethyl-hexylamine and n-tridecylamine.

C₁-C₈-alkoxyamine usually stands for methoxyamine, ethoxyamine, n-propoxyamine, isopropoxyamine, n-butyloxyamine, n-pentyloxyamine, n-hexyloxyamine, n-heptyloxyamine, n-octyloxyamine, preferred for C₁-C₆-alkoxyamine such as methoxyamine, ethoxyamine, n-propoxyamine, isopropoxyamine, n-butyloxyamine, n-hexyloxyamine.

C₁-C₈-alkoxy-C₁-C₈-alkylamine usually stands for methoxymethylamine, ethoxymethylamine, n-propoxymethylamine, isopropoxymethylamine, n-butyloxymethylamine, n-pentyloxymethylamine, n-hexyloxymethylamine, n-heptyloxymethylamine, n-octyloxymethylamine, 2-methoxyethylamine, ethoxyethylamine, n-propoxyethylamine, isopropoxyethylamine, n-butyloxyethylamine, n-pentyloxyethylamine, n-hexyloxyethylamine, n-heptyloxyethylamine, n-octyloxyethylamine, 3-methoxy-n-propylamine, 3-ethoxypropylamine, 3-isopropoxypropylamine, 4-methoxy-n-butylamine, 5-methoxy-n-pentylamine, 6-methoxy-n-hexylamine, 7-methoxy-n-heptylamine, 8-methoxy-n-octylamine, preferred 2-methoxy-ethylamine.

Preferred (R₉)₂N-C₁-C₈-alkylamines are 2-(dimethylamino)-methylamine, 2-(dimethylamino)-ethylamine, 2-(diethylamino)-ethylamine, 2-(diisopropylamino)-ethylamine, 2-(n-propylamino)-ethylamine, 3-(dimethylamino)-propylamine, 3-(cyclohexylamino)-propylamine.

Preferred amines of formula (IIa) are aniline, benzylamine, 3-methylphenylamine (m-toluidine), 2,6-diethylaniline, 2-isopropylaniline, 4-acetamidoaniline, 1-phenylethylamine, 2-phenylethylamine, 2-phenoxyethyl, 5,6,7,8-tetrahydro-1-naphthylamine, 5,6,7,8-tetrahydro-2-naphthylamine.

Particularly preferred amines RNH₂ are benzylamine, aniline, toluidine, n-butylamine, 1-phenylethylamine, 2-phenylethylamine, 2-methoxyethylamine, n-octylamine, 2,6-diethylaniline, n-octylamine, 2-phenoxyethylamine.

As a rule, the molar ratio of amine RNH₂ to acid chloride (IVb), preferably 5-sulfonyl chloride-isophthalic acid chloride, is chosen in the range of 1:1 to 10:1, preferably 4:1 to 8:1.

The reaction of acid chloride (IVb), preferably 5-sulfonyl chloride-isophthalic acid chloride, with an amine RNH₂ can be carried out without a solvent or in the presence of a solvent.

In case a solvent is used, the weight ratio of solvent to acid chloride (IVb) generally is not critical according to observations today, however, preferably a range of 1:1 to 25:1, more preferably from 2:1 to 20:1 can be chosen.

As solvent aprotic polar solvents such as dimethylformamide, dimethylamine, N-methylpyrrolidone, linear or cyclic ethers such as tetrahydrofuran, propyleneglycoldimethylether, anisole and the like can be used, as well as non-polar solvents such as alkanes like n-heptane, or aromatic solvents like toluene or xylene, as well as protic and polar solvents such as water or alcohols, in particularly C₁-C₄-alkanols such as methanol, ethanol, n-, i-propanol, n-butanol, preferred are aprotic polar and non-polar solvents.

The reaction temperature usually depends on the chosen reactivity of the amine RNH₂ und as a rule can be chosen in the range between 0 and 100°C.

The invention further relates to a preferred embodiment, in which the reaction temperature is chosen in the range of 0 to 25°C, and wherein the solvent is water or t-butyl-methyl ether, the molar ratio of amine RNH₂ to acid chloride (IVb) is chosen in the range of 1:1 to 10:1, preferably 4:1 to 8:1, and the weight ratio of solvent to acid chloride (IVb) can be in the range of 1:1 to 25:1, more preferably from 2:1 to 20:1.

The invention further relates to another preferred embodiment, in which the reaction temperature is chosen in the range of 20 to 100°C, and wherein the solvent is a non-polar solvent such as toluene, the molar ratio of amine RNH₂ to acid chloride (IVb) is chosen in the range of 1:1 to 10:1, preferably 4:1 to 8:1, and the weight ratio of solvent to acid chloride (IVb) can be in the range of 1:1 to 25:1, more preferably from 2:1 to 20:1.

The invention further relates to another preferred embodiment, in which the reaction temperature is chosen in the range of 0 to 100°C, and wherein the solvent is ethanol, the molar ratio of amine RNH₂ to acid chloride (IVb) is chosen in the range of 1:1 to 10:1, preferably 4:1 to 8:1, and the weight ratio of solvent to acid chloride (IVb) can be in the range of 1:1 to 25:1, more preferably from 2:1 to 20:1.

If desired the compounds of formula (I) obtained can be isolated or worked-up by known methods such as filtration, decantation, centrifugation, and optionally further procedures such as a washing step or (re-)crystallization.

If further desired, the washing step could be performed with water or acidified water, in example acidified with an inorganic acid such as hydro chloride acid, preferably having a pH in the range of 1 to 5.

According to observations up to now, the reactivity of the two functional carbonyl chloride groups of the acid chloride (IVb), in particular of 5-sulfonyl chloride-isophthalic acid dichloride, is higher compared to the sulfonyl chloride group. As a consequence, it is possible to synthesize products (I) with different groups R and R₁ by reacting the acid chloride (IVb), preferably 5-sulfonyl chloride-isophthalic acid dichloride, with a first amine R₁NH₂ followed by the reaction with a second amine RNH₂.

Therefore, a further preferred embodiment of this invention relates to the process for the manufacture of a compound with formula (I), wherein R and R₁ are different, comprising the steps of preparing a compound of formula (V), 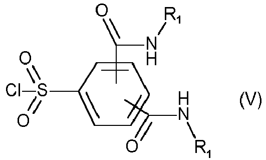 in particular of a compound of formula (Va), 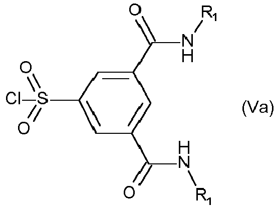 the preparation comprising
a) reacting an acid chloride of formula (IVb), preferably 5-sulfonyl chloride-isophthalic acid dichloride, with a first amine R₁NH₂,
b) reacting the obtained compound (V), preferably compound (Va), with a second amine RNH₂.

As a first amine R₁NH₂ ammonia, C₁-C₁₈-alkylamine, C₁-C₈-alkoxy-C₁-C₈-alkylamine, (R₉)₂N-C₁-C₈-alkylamine or an amine of formula (IIa) can be chosen.

Preferred amines R₁NH₂ can be benzylamine, aniline, toluidine, n-butylamine, 1-phenylethylamine, 2-phenylethylamine, 2-methoxyethylamine, n-octylamine, 2,6-diethylaniline, n-octylamine, 2-phenoxyethylamine.

Preferred second amines RNH₂ (not being the same as the first amine RNH₂) can be benzylamine, aniline, toluidine, n-butylamine, 1-phenylethylamine, 2-phenylethylamine, 2-methoxyethylamine, n-octylamine, 2,6-diethylaniline, n-octylamine, 2-phenoxyethylamine.

A further preferred embodiment of the instant invention relates to the above process for the manufacture of a compound with formula (I), wherein R and R₁ are different, comprising the steps of preparing a compound of formula (V), in particular of a compound of formula (Va), the preparation comprising
a) reacting an acid chloride of formula (IVb), preferably 5-sulfonyl chloride-isophthalic acid dichloride, with a first amine R₁NH₂,
b) reacting the obtained compound (V), preferably compound (Va), with a second amine RNH₂, preferably wherein the following combinations of first amine R₁NH₂ / second amine RNH₂ can be chosen: benzylamine/aniline, aniline/benzylamine, benzylamine/toluidine, n-butylamine/toluidine, 1-phenylethylamine/toluidine, 2-phenylethylamine/toluidine, 2-methox-yethylamine/toluidine, n-octylamine/toluidine, benzylamine/2,6-diethylaniline, n-octylamine/2,6-diethylaniline, 2-phenoxyethylamine/2,6-diethylaniline.

Generally, the molar ratio of the acid chloride (IVb), preferably of 5-sulfonyl chloride-isophthalic acid dichloride, to the first amine R₁NH₂ is chosen in the range of from 0.05:1 to 1:1, preferred from 0.1:1 to 0.4:1.

If desired, an additional base could be added in order to deprotonate the first amine R₁NH₂ such as inorganic bases like alkali metal hydroxides, earth alkali metal hydroxides, earth alkali metal carbonates such as NaOH, KOH, Ca(OH)₂, CaCO₃, or organic bases like triethylamine. Usually, such bases can be added in a molar ratio of additional base to R₁NH₂ in the range of from 1:1 to 10:1.

As a rule, the molar ratio of compound (V), preferably of compound (Va), to the second amine RNH₂ can be chosen in the range of from 0.1:1 to 1:1, preferred from 0.1:1 to 0.5:1.

The reaction of acid chloride (IVb), preferably 5-sulfonyl chloride-isophthalic acid chloride, with amines RNH₂ and R₁NH₂ can be carried out without a solvent or in the presence of a solvent.

In case a solvent is used, the weight ratio of solvent to acid chloride (IVb) generally is not critical according to observations today, however, preferably a range of 1:1 to 25:1, more preferably from 2:1 to 20:1 can be chosen.

As solvent aprotic polar solvents such as dimethylformamide, dimethylamine, N-methylpyrrolidone, linear or cyclic ethers such as tetrahydrofuran, propyleneglycoldimethylether, anisole and the like can be used, as well as non-polar solvents such as alkanes like n-heptane, or aromatic solvents like toluene or xylene, as well as protic and polar solvents such as water or alcohols, in particularly C₁-C₄-alkanols such as methanol, ethanol, n-, i-propanol, n-butanol, preferred are aprotic polar and non-polar solvents.

The reaction temperature of both reaction steps usually depends on the chosen reactivity of the amines RNH₂ and R₁NH₂ as well as of the chosen solvent and as a rule can be chosen in the range between 0 and 100°C.

In a preferred embodiment an aprotic solvent is chosen such as an aromatic solvent like benzene, toluene, xylene(s), anisole, chlorobenzene, o-dichlorobenzene, preferably toluene, the reaction temperature can be chosen as high as the boiling point of the reaction mixture, preferably in the range of from 20 to 100°C.

A further embodiment relates to the above process, wherein the reaction temperature of the first step is chosen in the range of from 60 to 100°C, preferably 80 to 100°C. A more preferred embodiment relates to the above process wherein the reaction temperature is chosen between 80 and 100°C and the molar ratio of the acid chloride (IVb), preferably of 5-sulfonyl chloride-isophthalic acid dichloride, to the first amine R₁NH₂ is chosen in the range of from 0.1:1 to 1:1, preferred from 0.2:1 to 0.5:1.

Another preferred embodiment of this invention related to the above process, wherein the reaction temperature of the first step is chosen in the range of from 20 to 60°C and the molar ratio of the acid chloride (IVb), preferably of 5-sulfonyl chloride-isophthalic acid dichloride, to the first amine R₁NH₂ is chosen in the range of from 0.05:1 to 1:1 preferred from 0.1:1 to 0.4:1

Usually, the reaction time for the first reaction step can be chosen in the range of from 1 hour to 10 hours, preferably from 2 to 7 hours. As a rule, the reaction time for the second reaction step can be chosen in the range of from 1 hour to 24 hours, preferably from 2 to 20 hours.

If desired the reaction mixture can be worked up by known methods, in example those mentioned above.

A further preferred embodiment of this invention relates to the process for the manufacture of compound (I), wherein R and R₁ have the same meaning, comprising the steps of
a) preparing an acid chloride of formula (IVb), preferably 5-sulfonyl chloride-isophthalic acid dichloride, the preparation comprising reacting a sulfo-isophthalic compound (IVa) 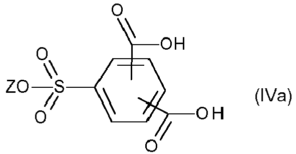 wherein Z stands for stands for hydrogen or an alkali metal, preferably a 5-sulfo-isophthalic compound (IV), with a chlorination agent,
b) optionally isolating the obtained acid chloride (IVb), preferably 5-sulfonyl chloride-isophthalic acid dichloride,
c) reacting the acid chloride (IVb), preferably 5-sulfonyl chloride-isophthalic acid dichloride, with an amine RNH2.

The reaction conditions can be chosen as described above.

A further preferred embodiment of this invention relates to the process for the manufacture of compound (I), wherein R and R₁ have a different meaning, comprising the steps of
a) preparing an acid chloride of formula (IVb), preferably 5-sulfonyl chloride-isophthalic acid dichloride, the preparation comprising reacting a sulfo-isophthalic compound (IVa) with a chlorination agent, as described above,
b) optionally isolating the obtained acid chloride (IVb), preferably 5-sulfonyl chloride-isophthalic acid dichloride, as described above,
c) reacting the acid chloride (IVb), preferably 5-sulfonyl chloride-isophthalic acid dichloride, with a first amine R₁NH₂, to obtain a compound of formula (V), preferably a compound of formula (Va),
d) reacting the obtained compound (V), preferably compound (Va), with a second amine RNH₂.

The reaction conditions can be chosen as described above.

Another preferred embodiment of this invention relates to the use of compound (I) as color developer in a heat sensitive recording material.

Methods for manufacturing heat sensitive coating compositions are well known in the art according to conventional methods. For example, at least one color forming compound and at least one color developer, optionally at least one sensitizer, optionally one stabilizer and optionally one pigment are milled separately or together in water or a suitable compound with dispersing capabilities by means of a mill such as a bead mill, an attritor, a sand mill or like milling apparatus to form an aqueous or other dispersion with an average particle diameter in the range of 0.1 to 2.0 µm, preferably from 0.3 to 1.0 µm.

Examples of suitable compounds with dispersing capabilities are water-soluble polymers such as polyvinyl alcohols, carboxylic acid-modified polyvinyl alcohols, sulfonic acid-modified polyvinyl alcohols, methyl cellulose, hydroxypropyl cellulose and hydroxypropylmethyl cellulose; anionic surfactants such as sodium salts of sulfonated naphthalene-formaldehyde condensation products, polyoxyethylene alkyl ether sulfuric acid ester salts and dialkylsulfosuccinic acid ester sodium salts; nonionic surfactants such as polyoxyethylene alkyl ethers and polyoxyethylene sorbitan fatty acid esters, or mixtures thereof, preferably polyvinyl alcohols, sulfonic acid-modified polyvinyl alcohols and methyl cellulose, or mixtures thereof.

The fine particle dispersions thus obtained are combined and then mixed with conventional amounts of binder, pigment, lubricant and, if desired, a stabilizer and/or one or more auxiliaries, and the resulting mixture is stirred to obtain a heat sensitive recording layer composition. The coating composition is applied to a support and the resulting coating is dried. The system of the invention can be employed for other end use applications using color forming materials, for example, a temperature indicating material.

The support can be a variety of suitable supports used in this field, and examples thereof include paper, wood-free paper made from non-chlorine bleached pulp, base paper containing waste paper plastic films, and synthetic paper.

Typically, the amount of the color former (or mixture of color formers) is chosen in the range of from 5 to15 % by weight, based on dry weight of heat sensitive coating composition.

Typically, the amount of the color developer (or mixture of color developers) is chosen in the range of from 10 to 50% by weight, based on dry weight of heat sensitive coating composition.

Another preferred embodiment of this invention relates to a heat sensitive recording material further comprising at least one sensitizer.

A particularly preferred embodiment relates to a heat sensitive recording material comprising as color former 3-dibutylamino-6-methyl-7-anilnofluoran, as color developer: 5-(N-3-methylphenyl-sulfonylamido)-(N',N"-bis-(3-methylphenyl)-isophthalic acid-diamide and as sensitizer benzyl-2-naphthyl ether.

Typically, the amount of the sensitizer (or mixture of sensitizers) is chosen in the range of from 10 to 50 % by weight, based on dry weight of heat sensitive coating composition.

Another preferred embodiment of this invention relates to a heat sensitive recording material further comprising at least one stabilizer.

A further particularly preferred embodiment relates to a heat sensitive recording material comprising as color former 3-dibutylamino-6-methyl-7-anilnofluoran, as color developer: 5-(N-3-methylphenyl-sulfonylamido)-(N',N"-bis-(3-methylphenyl)-isophthalic acid-diamide and as stabilizer carbamic acid, N,N-[sulfonylbis[4,1-phenyleneiminocarbonylimino(methylphenylene]]bis-C,C-diphenyl ester.

A further particularly preferred embodiment relates to a heat sensitive recording material comprising as color former 3-dibutylamino-6-methyl-7-anilnofluoran, as color developer: 5-(N-3-methylphenyl-sulfonylamido)-(N',N"-bis-(3-methylphenyl)-isophthalic acid-diamide, as sensitizer benzyl-2-naphthyl ether and as stabilizer carbamic acid, N,N-[sulfonylbis[4,1-phenyleneiminocarbonylimino(methylphenylene]]bis-C,C-diphenyl ester.

Typically, the amount of the stabilizer is chosen in the range of from 5 to 20 % by weight, based on dry weight of heat sensitive coating composition.

The color forming compounds are, for example, triphenylmethanes, lactones, or preferably fluorans.

Preferred color formers include but are not limited to: 3-diethylamino-6-methylfluoran, 3-dimethylamino-6-methyl-7-anilinofluoran, 3-diethylamino-6-methyl-7-anilinofluoran, 3-diethylamino-6-methyl-7-(2,4-dimethylanilino) fluoran, 3-diethylamino-6-methyl-7-chlorofluoran, 3-diethylamino-6-methyl-7-(3-trifluoromethylanilino) fluoran, 3-diethylamino-6-methyl-7-(2-chloroanilino) fluoran, 3-diethylamino-6-methyl-7-(4-chloroanilino) fluoran, 3-diethylamino-6-methyl-7-(2-fluoroanilino) fluoran, 3-diethylamino-6-methyl-7-(4-n-octylanilino) fluoran, 3-diethylamino -7-(4-n-octylanilino) fluoran, 3-diethylamino -7-(n-octylamino) fluoran, 3-diethylamino -7-(dibenzylamino) fluoran, 3-diethylamino-6-methyl-7-(dibenzylamino) fluoran, 3-diethylamino-6-chloro-7-methylfluoran, 3-diethylamino-7-t-butylfluoran, 3-diethylamino -7-carboxyethylfluoran, 3-diethylamino-6-chloro-7-anilinofluoran, 3-diethylamino-6-methyl-7-(3-methylanilino) fluoran, 3-diethylamino-6-methyl-7-(4-methylanilino) fluoran, 3-diethylamino-6-ethoxyethyl-7-anilinofluoran, 3-diethylamino-7-methylfluoran, 3-diethylamino-7-chlorofluoran, 3-diethylamino-7-(3-trifluoromethylanilino) fluoran, 3-diethylamino-7-(2-chloroanilino) fluoran, 3-diethylamino-7-(2-fluoroanilino) fluoran, 3-diethylamino-benzo[a] fluoran, 3-diethylamino-benzo[c] fluoran, 3-dibutylamino-7-dibenzylaminofluoran , 3-dibutylamino-7-anilinofluoran , 3-diethylamino-7-anilinofluoran, 3-dibutylamino-6-methyl fluoran, 3-dibutylamino-6-methyl-7-anilinofluoran, 3-dibutylamino-6-methyl-7-(2,4-dimethylanilino) fluoran, 3-dibutylamino-6-methyl-7-(2-chloroanilino) fluoran, 3-dibutylamino-6-methyl-7-(4-chloroanilino) fluoran, 3-dibutylamino-6-methyl-7-(2-fluoroanilino) fluoran, 3-dibutylamino-6-methyl-7-(3-trifluoromethylanilino) fluoran, 3-dibutylamino-6-ethoxyethyl-7-anilinofluoran, 3-dibutylamino-6-chloro-anilinofluoran, 3-dibutylamino-6-methyl-7-(4-methylanilino) fluoran, 3-dibutylamino-7-(2-chloroanilino) fluoran, 3-dibutylamino-7-(2-fluoroanilino) fluoran, 3-dibutylamino-7-(N-methyl-N-formylamino) fluoran, 3-dipentylamino-6-methyl-7-anilinofluoran, 3-dipentylamino-6-methyl-7-(2-chloroanilino) fluoran, 3-dipentylamino-7-(3-trifluoromethylanilino) fluoran, 3-dipentylamino-6-chloro-7-anilinofluoran, 3-dipentylamino-7-(4-chloroanilino) fluoran, 3-pyrrolidino-6-methyl-7-anilinofluoran, 3-piperidino-6-methyl-7-anilinofluoran, 3-(N-methyl-N-propylamino)-6-methyl-7-anilinofluoran, 3-(N-methyl-N-cyclohexylamino)-6-methyl-7-anilinofluoran, 3-(N-ethyl-N-cyclohexylamino)-6-methyl-7-anilinofluoran, 3-(N-ethyl-p-toluidino)-6-methyl-7-anilinofluoran, 3-(N-ethyl-N-isoamylamino)-6-methyl-7-anilinofluoran, 3-(N-ethyl-N-isoamylamino)-6-chloro-7-anilinofluoran, 3-(N-ethyl-N-tetrahydrofurfurylamino)-6-methyl-7-anilinofluoran, 3-(N-ethyl-N-isobutylamino)-6-methyl-7-anilinofluoran, 3-(N-butyl-N-isoamylamino)-6-methyl-7-anilinofluoran, 3-(N-isopropyl-N-3-pentylamino)-6-methyl-7-anilinofluoran, 3-(N-ethyl-N-ethoxypropylamino)-6-methyl-7-anilinofluoran, 3-cyclohexylamino-6-chlorofluoran, 2-methyl-6-p-(p-dimethylaminophenyl)aminoanilinofluoran, 2-methoxy-6-p-(p-dimethylaminophenyl)-aminoanilinofluoran, 2-chloro-3-methyl-6-p-(p-phenylaminophenyl)aminoanilinofluoran, 2-diethylamino-6-p-(p-dimethylaminophenyl)aminoanilinofluoran, 2-phenyl-6-methyl--6-p-(p-phenylaminophenyl)aminoanilinofluoran, 2-benzyl-6-p-(p-phenylaminophenyl)amino-anilinofluoran, 3-methyl-6-p-(p-dimethylaminophenyl)aminoanilinofluoran, 3-diethylamino-6-p-(p-diethylaminophenyl)aminoanilinofluoran, 3-diethylamino-6-p-(p-dibutylaminophenyl)-aminoanilinofluoran, 2,4-dimethyl-6-[(4-dimethylamino)-anilino] fluoran, 3-[(4-dimethyl-aminophenyl)amino]-5,7-dimethylfluoran, 3,6,6'-tris(dimethylamino)spiro[fluorene-9,3'-phthalide], 3,6,6'-tris(diethylamino)spiro[fluorene-9,3'-phthalide], 3,3-bis(p-dimethylamino-phenyl)-6-dimethylaminophthalide, 3,3-bis(p-dimethylaminophenyl)phthalide, 3,3-bis-[2-(p-dimethylaminophenyl)-2-(p-methoxyphenyl)ethenyl-4,5,6,7-tetrabromophthalide, 3,3-bis-[2-(p-dimethylaminophenyl)-2-(p-methoxyphenyl)ethenyl-4,5,6,7-tetrachlorophthalide, 3,3-bis[1,1-bis(4-pyrrolidinophenyl)ethylene-2-yl]-4,5,6,7-tetrabromophthalide, 3,3-bis-[1-(4-methoxyphenyl)-1-(4-pyrridinophenyl)ethylene-2-yl]-4,5,6,7-tetrachlorophthalide, 3-(4-diethylamino-2-ethoxyphenyl)-3-(1-ethyl-2-methylindole-3-yl)-4-azaphthalide, 3-(4-diethylamino-2-ethoxyphenyl)-3-(1-octyl-2-methylindole-3-yl)-4-azaphthalide, 3-(4-cyclohexylethylamino-2-methoxyphenyl)-3-(1-ethyl-2-methylindole-3-yl)-4-azaphthalide, 3,3-bis(1-ethyl-2-methylindole-3-yl) phthalide, 3,3-bis(1-octyl-2-methylindole-3-yl) phthalide, , 3-diethylamino-6,8-dimethylfluoran, 3-diethylamino-7,8-benzofluoran, 3-diethyl-aminofluoran-7-carboxylic acid ethyl ester, 3-[N-(4-methylphenyl)-N-ethylamino]-7-methylfluoran, and mixtures thereof.

All of the above color forming compounds can be used singly or as a mixture with other color forming compounds; or they may also be used together with further black color forming compounds.

Highly preferred are 3-dibutylamino-6-methyl-7-anilinofluoran, 3-dipentylamino-6-methyl-7-anilinofluoran, 3-(N-ethyl-N-isoamylamino)-6-methyl-7-anilinofluoran, 3-N-ethyl-p-toluidino-6-methyl-7-anilinofluoran, and mixtures thereof, particularly mixtures of 3-dibutylamino-6-methyl-7-anilinofluoran and 3-(N-ethyl-N-isoamylamino)-6-methyl-7-anilinofluoran.

In addition, the heat sensitive coating composition can contain a previously known developer, unless the color forming performance of the resultant heat sensitive material is disturbed thereby. Such developers are exemplified by but not limited to; 4,4'-isopropylidene bisphenol, 4,4'-sec-butylidene bisphenol, 4,4'-cyclohexylidene bisphenol, 2,2-bis-(4-hydroxyphenyl)-4-methylpentane, 2,2-dimethyl-3,3-di(4-hydroxyphenyl)butane, 2,2'-dihydroxydiphenyl, 1-phenyl-1,1-bis(4-hydroxyphenyl)butane, 4-phenyl-2,2-bis(4-hydroxyphenyl)butane, 1-phenyl-2,2-bis(4-hydroxyphenyl)butane, 2,2-bis(4'-hydroxy-3'-methylphenyl)-4-methylpentane, 2,2-bis(4'-hydroxy-3'-tert-butyllphenyl)-4-methylpentane, 4,4'-sec-butylidene-bis (2-methylphenol), 4,4'-isopropylidene-bis (2-tert-butylphenol), 2,2-bis(4'-hydroxy-3'-isopropylphenyl)-4-methylpentane, allyl-4,4-bis (4'-hydroxyphenyl) pentanoate, propargyl-4,4-bis(4'-hydroxyphenyl) pentanoate, n-propyl-4,4-bis (4'-hydroxyphenyl) pentanoate, 2,4-bis (phenylsulfonyl) phenol, 2-(4-methylsulfonyl)-4-(phenylsulfonyl) phenol, 2-(phenylsulfonyl)-4-(4-methylsulfonyl) phenol, 2,4-bis (4-methylphenylsulfonyl) phenol, pentamethylene-bis(4-hydroxybenzoate), 2,2-dimethyl-3,3-di(4-hydroxyphenyl)pentane, 2,2-di(4-hydroxyphenyl)hexane, 4,4'-dihydroxydiphenyl thioether, 1,7-di(4-hydroxyphenylthio)-3,5-dioxaheptane, 2,2'-bis(4-hydroxyphenylthio)diethyl ether, 4,4'-dihydroxy-3,3'-dimethylphenyl thioether; benzyl-4-hydroxybenzoate, ethyl-4-hydroxybenzoate, propyl-4-hydroxybenzoate, isopropyl-4-hydroxybenzoate, butyl-4-hydroxybenzoate, isobutyl-4-hydroxybenzoate, 4,4'-dihydroxydiphenyl sulfone, 2,4'-dihydroxydiphenyl sulfone, 4-hydroxy-4'-methyldiphenyl sulfone, 4-hydroxy-4'-isopropoxydiphenyl sulfone, 4-hydroxy-4'-butoxydiphenyl sulfone, 4-hydroxy-4'-benzyloxydiphenyl sulfone, 4,4'-dihydroxy-3,3'-diallyldiphenyl sulfone, 4-[[4-(2-propenyl-1-yloxy)phenol]sulfonyl phenol, 3,4-dihydroxy-4'-methyldiphenyl sulfone, 4,4'-dihydroxy-3,3',5,5'-tetrabromodiphenyl sulfone, 4,4'-bis (p-toluenesulphonylamino-carbonylamino) diphenylmethane, N-p-toluenesulphonyl-N'-phenyl urea, N-p-toluenesulfonyl-N'-3-(p-toluenesulfonyloxy)phenylurea, N-[2-(3-phenylureido)phenyl] benzenesulfonamide, N-phenyl-N'-[phenylamino)sulfonyl]urea, N-(4-methylphenyl)-N-[[4-methylphenyl)amino]sulfonyl]urea, N-[(phenylamino)sulfonyl]benzamide, dimethyl 4-hydroxyphthalate, dicyclohexyl 4-hydroxyphthalate, diphenyl 4-hydroxyphthalate, 4-[2-(4-methoxyphenyloxy)ethyloxy] salicylate, 3,5-di-tert-butylsalicylic acid, 3-benzyl salicylic acid, 3-(α-methylbenzyl) salicylic acid, 3-phenyl-5-(α,α-dimethylbenzyl) salicylic acid, 3,5-di-α-methylbenzyl salicylic acid; metal salts of salicylic acid, 2-benzylsulfonylbenzoic acid, 3-cyclohexyl-4-hydroxybenzoic acid, zinc benzoate, zinc 4-nitrobenzoate, 4-(4'-phenoxy-butoxy)phthalic acid, 4-(2'-phenoxyethoxy)phthalic acid, 4-(3'-phenylpropyloxy)phthalic acid, mono (2-hydroxyethyl) -5-nitro-isophthalic acid, 5-benzyloxycarbonyl isophthalic acid, 5-(1'-phenylethanesulfonyl) isophthalic acid, bis(1,2-dihydro-1,5-dimethyl-2-phenyl-3H-pyrazol-3-one-O)bis(thiocyanato-N) zinc, and mixtures thereof.

Representative examples of sensitizer are stearamide, methylol stearamide, methylene bis-stearamide, ethylene bis-stearamide, amide waxes, p-benzylbiphenyl, m-terphenyl, benzyl-2-naphthyl ether, 4-methoxybiphenyl, dibenzyl oxalate, di(4-methylbenzyl) oxalate, di(4-chlorobenzyl) oxalate, diphenyl sulfone, dimethyl terephthalate, dibenzyl terephthalate, dibenzyl isophthalate, 1,2-diphenoxyethane, 1,2-bis(4-methylphenoxy) ethane, 1,2-bis(3-methylphenoxy) ethane, 4,4'-dimethylbiphenyl, phenyl-1-hydroxy-2-naphthoate, 4-methylphenyl biphenyl ether, 1,2-bis(3,4-dimethylphenyl) ethane, 2,3,5,6-4'-methyldiphenyl methane, 1,4-diethoxynaphthalene, 1,4-diacetoxybenzene, 1,4-diproprionoxybenzene, o-xylylene-bis(phenyl ether), 4-(m-methylphenoxymethyl) biphenyl, p-hydroxyacetanilide, p-hydroxybutyranilide, p-hydroxynonananilide, p-hydroxylauranilide, p-hydroxyoctadecananilide, N-phenyl-phenylsulphonamide, acetyl biphenyl compounds (e.g. as described in JP2003 063149A2) and 2-phenoxyethyl-N-phenylcarbamate.

Preferred sensitizers are stearamide, amide waxes, benzyl-2-naphthyl ether, di(4-methylbenzyl) oxalate, diphenyl sulfone, 1,2-diphenoxyethane and 1,2-bis(3-methylphenoxy) ethane.

Representative stabilizers for use in the inventive method are selected from the group consisting of 2,2'-methylene-bis(4-methyl-6-tert-butylphenol), 2,2'-methylene-bis(4-ethyl-6-tert-butylphenol), 4,4'-butylidene-bis(3-methyl-6-tert-butylphenol), 4,4'-thio-bis(2-tert-butyl-5-methylphenol), 1,1,3-tris(2-methyl-4-hydroxy-5-tert-butylphenyl) butane, 1,1,3-tris(2-methyl-4-hydroxy-5-cyclohexylphenyl) butane, bis (3-tert-butyl-4-hydroxy-6-methylphenyl) sulfone, bis (3,5-dibromo-4-hydroxyphenyl) sulfone, 4,4'-sulfinyl bis (2-tert-butyl-5-methylphenol), 2,2'-methylene bis (4,6-di-tert-butylphenyl) phosphate and alkali metal, ammonium and polyvalent metal salts thereof, 4-benzyloxy-4'-(2-methylglycidyloxy) diphenyl sulfone, 4,4'-diglycidyloxydiphenyl sulfone, 1,4-diglycidyloxybenzene, 4-[α-(hydroxymethyl)benzyloxy]-4-hydroxydiphenyl sulfone, metal salts of p-nitrobenzoic acid, metal salts of phthalic acid mono benzyl ester, metal salts of cinnamic acid, 4,4'-sulfonyl bisphenol, polymer with 1,1'-oxybis[2-chloroethane] with CAS No. 191680-83-8, carbamic acid, N,N-[sulfonylbis[4,1-phenyleneiminocarbonylimino(methylphenylene]]bis-C,C-diphenyl ester with CAS No. 874187-71-0 and mixtures thereof.

Preferred stabilizers are 4,4'-butylidene-bis(3-methyl-6-tert-butylphenol), 4,4'-thio-bis(2-tert-butyl-5-methylphenol), 1,1,3-tris(2-methyl-4-hydroxy-5-tert-butylphenyl) butane, 1,1,3-tris(2-methyl-4-hydroxy-5-cyclohexylphenyl) butane, ,4'-sulfonyl bisphenol, polymer with 1,1'-oxybis[2-chloroethane] and carbamic acid, N,N-[sulfonylbis[4,1-phenyleneiminocarbonylimino(methylphenylene]]bis-C,C-diphenyl ester and mixtures thereof.

Representative binders for use in the heat sensitive recording layer include polyvinyl alcohol (fully or partially hydrolysed), carboxy-modified polyvinyl alcohol, acetoacetyl-modified polyvinyl alcohol, diacetone-modified polyvinyl alcohol, silicon-modified polyvinyl alcohol, sulfonic-modified polyvinyl alcohol, oxidised starch, gelatine, casein, derivatives of cellulose such as hydroxyethyl cellulose, methyl cellulose, ethyl cellulose, carboxymethyl cellulose and acetyl cellulose, starch-vinyl acetate graft copolymers, styrene-maleic anhydride copolymers, methyl vinyl ether-maleic anhydride copolymers, isopropylene-maleic anhydride copolymers and like water-soluble resins, styrene-butadiene latex, acrylic latex, urethane latex and like water-dispersible resins and mixtures thereof. The amount of the binder to be used is about 5 to 40 weight-%, preferably about 7 to 30 % by weight, based on the heat sensitive recording layer.

Representative pigments for use in the heat sensitive recording layer include ground calcium carbonate, precipitated calcium carbonate, kaolin, calcined kaolin, aluminium hydroxide, talc, titanium dioxide, zinc oxide, amorphous silica, barium sulfate, polystyrene resin, urea-formaldehyde resin, hollow plastic pigment and mixtures thereof. The amount of pigment to be used is about 5 to 75 weight-%, preferably about 10 to 60 weight-% based on the heat sensitive recording layer.

Representative lubricants for use in the heat sensitive recording layer include stearamide, methylene bis stearamide, polyethylene wax, carnauba wax, paraffin wax, zinc stearate, calcium stearate and mixtures thereof. The amount of lubricants to be used is usually in the range from 2 to 10 weight-%, preferably from 3 to 7 weight-%, based on the heat sensitive coating composition.

Examples of various auxiliaries that can be added to the heat sensitive layer coating composition, if so desired, include surfactants such as sodium dioctylsulfosuccinate, sodium dodecybenzenesulfonate, sodium lauryl sulfate and fatty acid metal salts; insolubilisers such as glyoxal, urea-formaldehyde resins, melamine-formaldehyde resins, polyamide resins, polyamideamine-epichlorohydrin resins, adipic acid dihydrazide, boric acid, borax, ammonium zirconium carbonate and potassium zirconium carbonate; antifoaming agents, fluorescent whitening agents, fluorescent dyes and/or pigments, tinting dyes and UV absorbers. The amount of auxiliaries to be used is usually chosen within the range of from 0.1 to 5 weight-%, based on the heat sensitive coating composition.

The ultraviolet absorbers may be employed in either the thermosensitive coloring layer or in a protective layer, and if desired, may be used in microencapsulated form in the protective layer.

Representative examples of ultraviolet absorbers that may be used in the invention include phenyl salicylate, p-tert-butylphenyl salicylate, p-octylphenyl salicylate and like salicylic acid type ultraviolet absorbers.

2,4-Dihydroxybenzophenone, 2-hydroxy-4-methoxybenzophenone, 2-hydroxy-4-octyloxybenzophenone, 2-hydroxy-4-dodecyloxybenzophenone, 2,2'-dihydroxy-4-methoxybenzophenone, 2,2'-dihydroxy-4,4'-dimethoxybenzophenone, 2-hydroxy-4-methoxy-5-sulfobenzophenone and like benzophenone type ultraviolet absorbers; 2-ethylhexyl-2-cyano-3,3-diphenylacrylate, ethyl-2-cyano-3,3-diphenylacrylate and like cyanoacrylate type ultraviolet absorbers; bis(2,2,6,6-tetramethyl-4-piperidyl) sebacate, bis(2,2,6,6-tetramethyl-4-piperidyl) succinate, bis(1,2,2,6,6-pentamethyl-4-piperidyl)-2-(3,5-di-tert-butyl-4-hydroxybenzyl)-2-n-butyl malonate and like hindered amine type ultraviolet absorbers;

2-(2'-Hydroxyphenyl) benzotriazole, 2-(2'-hydroxy-5'-methylphenyl) benzotriazole, 2-(2'-hydroxy-5'-tert-butylphenyl)benzotriazole, 2-(2'-hydroxy-3,5'-di-tert-butylphenyl) benzotriazole, 2-(2'-hydroxy-3'-tert-butyl-5'-methylphenyl)-5-chlorobenzotriazole, 2-(2'-hydroxy-3,5'-di-tert-butylphenyl)-5-chlorobenzotriazole, 2-(2'-hydroxy-3,5'-di-tert-butylphenyl)-5-tert-butylbenzotriazole, 2-(2'-hydroxy-3,5'-di-tert-amylphenyl) benzotriazole, 2-(2'-hydroxy-3,5'-di-tert-amylphenyl)-5-tert-amylbenzotriazole, 2-(2'-hydroxy-3,5'-di-tert-amylphenyl)-5-methoxybenzotriazole, 2-[2'-hydroxy-3'-(3",4",5",6"-tetrahydrophthalimido-methyl)-5'-methylphenyl] benzotriazole, 2-(2'-hydroxy-5'-tert-octylphenyl)benzotriazole, 2-(2'-hydroxy-3'-sec-butyl-5'-tert-butylphenyl) benzotriazole, 2-(2'-hydroxy-3'-tert-amyl-5'-phenoxyphenyl)-5-methylbenzotriazole, 2-(2'-hydroxy-5'-n-dodecylphenyl) benzotriazole, 2-(2'-hydroxy-5'-sec-octyloxyphenyl)-5-phenylbenzotriazole, 2-(2'-hydroxy-3'-tert-amyl-5'-phenylphenyl)-5-methoxybenzotriazole, 2-[2'-hydroxy-3',5'-bis(α,α-dimethylbenzyl)-phenyl] benzotriazole and like benzotriazole ultraviolet absorbers;

2-(2'-Hydroxy-3'-dodecyl-5'-methylphenyl) benzotriazole, 2-(2'-hydroxy-3'-undecyl-5'-methylphenyl) benzotriazole, 2-(2'-hydroxy-3'-tridecyl-5'-methylphenyl) benzotriazole, 2-(2'-hydroxy-3'-tetradecyl-5'-methylphenyl) benzotriazole, 2-(2'-hydroxy-3'-pentadecyl-5'-methylphenyl) benzotriazole, 2-(2'-hydroxy-3'-hexaadecyl-5'-methylphenyl) benzotriazole, 2-[2'-hydroxy-4'-(2"-ethylhexyl)oxyphenyl] benzotriazole, 2-[2'-hydroxy-4'-(2"-ethylheptyl)oxyphenyl] benzotriazole, 2-[2'-hydroxy-4'-(2"-ethyloctyl)oxyphenyl] benzotriazole, 2-[2'-hydroxy-4'-(2"-propyloctyl)oxyphenyl] benzotriazole, 2-[2'-hydroxy-4'-(2"-propylheptyl)oxyphenyl] benzotriazole, 2-[2'-hydroxy-4'-(2"-propylhexyl)oxyphenyl] benzotriazole, 2-[2'-hydroxy-4'-(1"-ethylhexyl)oxyphenyl] benzotriazole, 2-[2'-hydroxy-4'-(1 "-ethylheptyl)oxyphenyl] benzotriazole, 2-[2'-hydroxy-4'-(1"-ethyloctyl)oxyphenyl] benzotriazole, 2-[2'-hydroxy-4'-(1"-propyloctyl)oxyphenyl] benzotriazole, 2-[2'-hydroxy-4'-(1"-propylheptyl)oxyphenyl] benzotriazole, , 2-[2'-hydroxy-4'-(1"-propylhexyl)oxyphenyl] benzotriazole, 2-(2'-hydroxy-3'-sec-butyl-5'-tert-butylphenyl)-5-n-butylbenzotriazole, 2-(2'-hydroxy-3'-sec-butyl-5'-tert-butylphenyl)-5'-tert-pentylbenzotriazole, 2-(2'-hydroxy-3'-sec-butyl-5'-tert-butylphenyl)-5-n-pentylbenzotriazole, 2-(2'-hydroxy-3'-sec-butyl-5'-tert-pentylphenyl)-5'-tert-butylbenzotriazole, 2-(2'-hydroxy-3'-sec-butyl-5'-tert-pentylphenyl)-5'-n-butylbenzotriazole, 2-(2'-hydroxy-3',5'-di-tert-butylphenyl)-5-sec-butylbenzotriazole, 2-(2'-hydroxy-3',5'-di-tert-pentylphenyl)-5-sec-butylbenzotriazole, 2-(2'-hydroxy-3'-tert-butyl-5'-tert-pentylphenyl)-5-sec-butylbenzotriazole, 2-(2'-hydroxy-3,5'-di-sec-butylphenyl)-5-chlorobenzotriazole, 2-(2'-hydroxy-3,5'-di-sec-butylphenyl)-5-methoxybenzotriazole, 2-(2'-hydroxy-3,5'-di-sec-butylphenyl)-5-tert-butylbenzotriazole, 2-(2'-hydroxy-3,5'-di-sec-butylphenyl)-5-n-butylbenzotriazole, octyl-5-tert-butyl-3-(5-chloro-2H-benzotriazole-2-yl)-4-hydroxybenzene propionate, condensate of methyl -3-[tert-butyl-5-(2H-benzotriazole-2-yl)-4-hydroxyphenyl] propionate and polyethylene glycol (molecular weight: about 300) and like benzotriazole ultraviolet absorbers.

The heat sensitive recording layer coating composition is applied to the support in an amount of about 1 to 10 g/m², preferably about 3 to 7 g/m² on a dry weight basis. The heat sensitive recording layer coating composition may be applied to the support by a known coating device such as a coating bar, a roll coater, an air knife coater, a blade coater, a gravure coater, a die coater or a curtain coater.

If desired, an undercoat layer can also be provided between the support and the heat sensitive recording layer in order to improve the thermal sensitivity and efficiency during recording. The undercoat layer is formed by coating the support with an undercoat layer coating composition comprising as main components organic hollow particles and/or an oil absorbing pigment and a binder and then drying the coating.

Representative examples of oil absorbing pigments include kaolin, calcined kaolin, amorphous silica, precipitated calcium carbonate and talc. The average pigment diameter may be in the range 0.01 to 5 µm, preferably from 0.02 to 3 µm.

Representative examples of organic hollow particles include particles having a shell made from an acrylic resin, styrene-based resin and vinylidene chloride-based resin and having a void ratio of about 50 to 99 %. The outside diameter of the organic hollow particle may be in the range 0.5 to 10 µm, preferably from 1 to 5 µm.

The organic hollow particles may be expandable hollow particles. A typical example of such expandable hollow particles are microcapsules having an average diameter of 0.1 to 5 µm comprising a vinylidene chloride resin shell and butane gas as fill material. When a support coated with an undercoat layer comprising such expandable hollow particles is subjected to heat treatment, the microcapsules expand to an average particle diameter of 1 to 30 µm.

When the oil absorbing pigment is used in combination with the organic hollow particles, the combined amount of the two components is preferably about 40 to 90 weight-%, particularly about 50 to 80 weight-% based on the undercoat layer.

The binder used in the undercoat layer usually is selected from the binders to be used in the heat sensitive recording layer and particularly preferred examples are styrene-butadiene latex, a polyvinyl alcohol or starch-vinyl acetate copolymer. The amount of binder to be used usually is in the range of about 5 to 30 weight-%, particularly 10 to 20 weight-%, based on the undercoat layer.

As a rule, the undercoat recording layer coating composition is applied to the support in an amount of about 2 to 20 g/m², preferably about 4 to 12 g/m² on a dry weight basis.

If desired, a protective layer may be provided on the heat sensitive recording layer to enhance the resistance of the recorded image to water and chemicals, for example, oils, fats, alcohols, plasticisers and the like to improve the runability during recording. Generally, the protective layer is formed by coating the heat sensitive recording layer with a protective layer coating composition comprising as main components a binder having film-forming ability and optionally, a pigment and/or an insolubiliser and/or a lubricant and then drying the resulting coating film.

Representative examples of the binder to be used in the protective layer coating composition include polyvinyl alcohol (fully or partially hydrolysed), carboxy-modified polyvinyl alcohol, acetoacetyl-modified polyvinyl alcohol, diacetone-modified polyvinyl alcohol, silicon-modified polyvinyl alcohol, starches, gelatine, casein, gum arabic, derivatives of cellulose such as hydroxyethyl cellulose, methyl cellulose, ethyl cellulose, carboxymethyl cellulose and acetyl cellulose, starch vinyl acetate graft copolymers, styrene-maleic anhydride copolymers, methyl vinyl ether-maleic anhydride copolymers, isopropylene-maleic anhydride copolymers and like water-soluble resins, styrene-butadiene latex, acrylic latex, urethane latex and like water-dispersible resins and mixtures thereof.

Pigment, insolubiliser, lubricant and, if required, other auxiliaries may be selected from those used in the heat sensitive recording layer coating composition as described above.

Usually, the protective layer coating composition is applied in an amount of about 0.5 to 10 g/m², preferably about 1 to 5 g/m² on a dry weight basis and may be applied with a similar coating device to that used to coat the heat sensitive layer.

It is also possible to provide a protective layer, an adhesive layer and a magnetic layer on the rear side of the support.

The following non-limiting examples illustrate the novel materials of the present invention.

### Examples:

### Example 1: Synthesis of 5-sulfonylchloride-isophthalic acid dichloride

To a mixture of 42 g (150 mmol) of 5-sulfo-isophthalic acid sodium salt (95%, Sigma-AI-drich Inc.) and 160 ml (2200 mmol) thionyl chloride (>99% GC, Fluka), 10 ml of N,N-dimethylformamide were added under stirring. The suspension was slowly heated to reflux and kept at reflux conditions for two hours during which the formation of HCI and SO₂ indicated the progress of the reaction. After the formation of gas ceased, the reaction mixture was cooled down to room temperature. The yellowish suspension obtained was poured slowly onto 1000 g of ice flakes under stirring during which the slightly pinkish product precipitated. After stirring for 30 min at below 5°C the precipitate was filtered. The thus obtained wet filter cake was deep frozen and freeze-dried in order to avoid premature hydrolysis. Yield: 45.5 g slightly yellowish powder. NMR (d6-DMSO): 9.15 (1H), 9.04 (2H).

### Example 2: 5-(N-Benzyl-sulfonylamido)-(N',N"-dibenzyl)-isophthalic acid-diamide (water) (Method A)

15 g (50 mmol) 5-sulfonylchloride-isophthalic acid dichloride (as synthesized in example 1) were suspended in 125 ml of demineralized water (having a temperature in the range of 0 to 5°C). Under stirring 32.7 g (310 mmol) benzylamine (≥99%, Sigma-Aldrich Inc.) were slowly added during 20 minutes at a temperature in the range of 0 to 5°C. After the addition of benzylamine a white suspension formed which was further stirred for 22 hours. Then 100 ml of water were added to the suspension, the precipitate obtained was filtered, afterwards washed with 500 ml water and subsequently with 150 ml of hydrochloric acid (10%). The thus obtained filter cake was dried under reduced pressure (200 mbar) at 50°C.
Yield: 20.5g (86%) of a white powder, melting point: 190°C.

### Example 3: 5-(N-Benzyl-sulfonylamido)-(N',N"-dibenzyl)-isophthalic acid-diamide (t-Butyl-methylether) (Method B)

3 g (10 mmol) 5-Sulfonylchloride-isophthalic acid dichloride (as synthesized in example 1) were dissolved in 15 ml t-butyl-methylether. The clear solution was stirred for 15 min at room temperature and cooled down to a temperature in the range of 0 to 5°C. Under stirring a solution of 4.37 g benzylamine (≥99%, Sigma-Aldrich Inc.) in t-butyl-methylether was added during 60 min. A white precipitate formed instantly. After further stirring for 1 h the temperatur was increased to room temperature. The precipitate was filtered off and washed subsequently with 30 ml t-butylmethylether, 50 ml water and 30 ml HCl (10%). The white product was dried under a reduced pressure (200 mbar / 50°C) overnight.
Yield: 4.18 g (82%) of a white powder

### Example 4: 5-(N-3-Methylphenyl-sulfonylamido)-(N',N"-bis-(3-methylphenyl)-isophthalic acid-diamide (toluene) (Method C)

1.5 g (5 mmol) 5-Sulfonylchloride-isophthalic acid dichloride (as synthesized in example 1) were dispersed in 20 ml toluene at room temperature. To the white dispersion 3.3 g (30.9 mmol) 3-methyl-aniline were slowly added. A further 60 ml toluene were added to the crude suspension which was then heated to 100°C and stirred for 18 h. After cooling to room temperature the precipitate was filtered and washed with 30 ml toluene, followed by the addition of 50 ml demineralized water and 30 ml HCI (10%). The compound was dried under reduced pressure (200 mbar) at 40°C.
Yield: 2 g of a reddish product

### Recrystallisation

10 g raw 5-(N-3-methylphenyl-sulfonylamido)-(N',N"-bis-(3-methylphenyl)-isophthalic acid-diamide obtained in example 4 were dissolved in 350 ml methanol under reflux conditions resulting in a clear solution. 35 ml water were added after which some slight haze could be observed. The mixture was slowly cooled to room temperature during 2 h. The white precipitated needles were filtered off, washed with 50 ml of demineralized water and dried at 50°C under reduced pressure (200mbar).
Yield: 52%.
Melting point (DSC, 4°C/min): 195.3°C, 215.2°C, (different crystal modifications)
NMR (d₆-DMSO): 2.20 (3H), 2.32 (6H), 6.85 (1H), 6.92 (1H), 6.96 (3H), 7.11 (1H), 7.26 (2H), 7.58 (4H), 8.46 (2H), 8.73 (1H), 10.45 (1H), 10.53 (2H)

### Example 4a: Directed synthesis of the α-modification of 5-(N-3-methylphenyl-sulfonyl-amido)-(N',N"-bis-(3-methylphenyl)-isophthalic acid-diamide

To a mixture of 49.0 g (0.16 mol) of 5-sulfonylchloride-isophthalic acid dichloride (as synthesized in example 1) and 500 ml of toluene at 22°C, 110 ml (0.99 mol) m-toluidine were added dropwise within 30 minutes. Then, again additional 500 ml toluene were added. The product mixture obtained was heated to 100°C. Then 1500 ml of toluene were added and the reaction mixture was kept at 100°C for 3 hours. Afterwards, the reaction mixture was allowed to cool to 22°C. The precipitate formed was filtered off. The filter cake obtained was suspended in 250 ml of water under stirring. This suspension was heated to 80°C for 30 min. The aqueous layer was removed by decantation at 80°C. Then 200 ml of 10% by weight hydro chloric acid were added to the residue and the mixture was stirred for 30 min at 40°C. Then, the aqueous layer was decanted once more. Thereafter, 800 ml of n-heptane were added to the residue and stirred for 30 minutes at 22°C. The precipitate was collected by filtration and dried under the reduced pressure of a vacuum pump at 60°C.
Yield: 81 g (96%), white solid, m.p. 211.2°C (DSC)

### Example 4b: Directed synthesis of the β-modification of 5-(N-3-methylphenyl-sulfonyl-amido)-(N',N"-bis-(3-methylphenyl)-isophthalic acid-diamide

To a mixture of 10.8 g (0.1 mol) of m-toluidine in 50 ml tetrahydrofuran at 22°C a solution of 5.0 g (0.0166 mol) 5-sulfonylchloride-isophthalic acid dichloride (as synthesized in example 1) in 15 m tetrahydrofuran were added dropwise within 15 min under stirring. The reaction mixture was then heated to 65°C for 5 hours. The formed precipitate was removed by filtration. The filtrate was dried under the reduced pressure of a vacuum pump. The obtained residue was taken up into 25 ml tetrahydrofuran. The solution obtained was then poured into 100 ml of a methanol/water (9:1) mixture and heated to 50°C for one hour. Afterwards, the reaction mixture was cooled down to 22°C. The solid obtained was collected by filtration and washed with methanol. The product was then dried under reduced pressure at 60°C for 7 hours.
Yield: 5.0 g (58%), white solid, m.p. 192.2°C (DSC)

### Example 4c: Directed synthesis of the γ-modification of 5-(N-3-methylphenyl-sulfonyl-amido)-(N',N"-bis-(3-methylphenyl)-isophthalic acid-diamide

To a mixture of 10.8 g (0.1 mol) m-toluidine in 75 ml n-heptane, 5 g (0.0166 mol) 5-sulfonylchloride-isophthalic acid dichloride (as synthesized in example 1) in 20 ml toluene were added dropwise within 45 minutes at 75°C. The reaction mixture was then heated to 90°C for 5 hours, then cooled to 60°C. Subsequently, 50 ml of water followed by 5 ml of concentrated hydrochloric acid were added at this temperature, and then stirred for another 15 minutes at 60°C. Afterwards, the solid phase obtained was filtered off at this temperature. Thereafter, the filter cake obtained was washed first with 50 ml of water, then with 50 ml of n-heptane and subsequently dried on a suction filter by sucking air through the filter using a vacuum pump for 1 hour. The thus dried filter cake was suspended in 90 ml of a methanol/water mixture (9:1) and the suspension was stirred for two hours at 60°C. After cooling the suspension to 20°C, the precipitate obtained was filtered off and washed with 50 ml of a methanol/water mixture (1:1). Afterwards, the product was dried under the reduced pressure of a vacuum pump at 60°C for 7 hours.
Yield: 7.0 g (white solid) 82%, m.p. 215.6 °C, determined by DSC.

### Example 5: 5-(N-2,6-Diethylphenyl-sulfonylamido)-(N',N"-bis-(2,6-diethylphenyl)-isophthalic acid-diamide (ethanol) (Method D)

To a mixture of 1.5 g (5 mmol) 5-sulfonylchloride-isophthalic acid dichloride (as synthesized in example 1) and 25 ml ethanol cooled down to a temperature in the range of 0 to 5°C 2.4 g (16.1 mmol) 2,6-diethylaniline were added slowly under stirring. After 1 h stirring at a temperature in the range of 0 to 4°C the yellow-grey suspension was heated to the boiling point under reflux and kept there for 30 min during which the color changed to beige-red. The reaction mixture was then cooled to 10°C, the precipitate obtained filtered and washed with 20 ml of ethanol.
Yield: 0.3 g of colorless needles
From the filtrates, a further 1 g of colorless needles could be isolated by evaporation of the solvent.
Combined yield: 1.3 g (41%) 5-(N-2,6-diethylphenyl-sulfonylamido)-(N',N"-bis-(2,6-diethylphenyl)-isophthalic acid-diamide
Mp: >210°C

The following products were obtained as mentioned above:

| **Examples** | **Substituent R=R₁** | **Method** | **Mp (°C)** | **Yield (%)** |
|---|---|---|---|---|
| 6 | phenyl | C | 187- >200 | 85 |
| 7 | o-i-propyl-phenyl- | C | >200 | 42 |
| 8 | p-acetamido-phenyl- | C | >200 | 90 |
| 9 | 1-tetralino- | B | 134-140 | 98 |
| 10 | 1-phenylethyl- | B | 120-123 | 79 |
| 11 | 2-phenylethyl- | B | 140-141 | 85 |
| 12 | 2,6-diethylphenyl- | C | >210 | 66 |
| 13 | butyl- | A | 120 | 64 |
| 14 | 2-ethyl-hexyl- | A | nm | 57 |

| | | | | |
|---|---|---|---|---|
| nm: not measured | | | | |

### Example 15: 5-(N-Benzyl-sulfonylamido)-(N',N"-diphenyl-isophthalic acid-diamide (toluene) (Method F)

To a mixture of 1.5 g (5 mmol) 5-sulfonylchloride-isophthalic acid dichloride (as synthesized in example 1) in 25 ml toluene at 80°C 1.06 g (11 mmol) of aniline were slowly added. After 5 h of stirring at a temperature in the range of 85 to 90°C the white suspension obtained was cooled to room temperature. Then 1.7 g (15.9 mmol) benzylamine were added and the reaction mixture was stirred for a further 17 h at a temperature in the range of 60 to 65°C. After cooling to room temperature, the precipitate obtained was filtered and subsequently washed with 30 ml toluene, followed by 50 ml demineralized water and 30 ml of HCI (10%). The wet filter cake was dried under reduced pressure (200mbar) at 40°C.
Yield: 1.7 g (70%) white powder of 5-(N-benzyl-sulfonylamido)-(N',N"-bisphenyl-isophthalic acid-diamide

The following asymmetrically substituted products were obtained as mentioned above:

| **Examples** | **R** | **R₁** | **Yield (%)** |
|---|---|---|---|
| 16 | phenyl- | benzyl- | 56 |
| 17 | benzyl- | 3-methyl-phenyl- | 71 |
| 18 | butyl- | 3-methyl-phenyl- | 82 |
| 19 | 1-phenyl-ethyl- | 3-methyl-phenyl- | 72 |
| 20 | 2-phenyl-ethyl- | 3-methyl-phenyl- | 79 |
| 21 | 2-methoxy-ethyl- | 3-methyl-phenyl- | 96 |
| 22 | octyl- | 3-methyl-phenyl- | 95 |
| 23 | benzyl- | 2,6-diethylphenyl- | 100 |
| 24 | octyl- | 2,6-diethylphenyl- | 64 |
| 25 | 2-phenoxy-ethyl- | 2,6-diethylphenyl- | 88 |

### Application examples

The following non-limiting examples illustrate the application of the novel materials of the present invention.

### Milling example 1:

### Preparation of color developer dispersion A-1 comparative

A mixture of 5 g of N-p-toluenesulfonyl-N'-3-(p-toluenesulfonyloxy)phenylurea (Pergafast 201, BASF SE), 0.1 g of a dispersing agent (sodium salt of naphthalene sulfonic acid condensation product with formaldehyde, TAMOL® NN 9401 from BASF SE), 3.4 g of a 10% by weight solution of polyvinyl alcohol (Mowiol® 40-88, Mw∼ 205.000 g/mol, Sigma-Aldrich Inc. / Kuraray Europe GmbH) and 4 g of water was milled in a bead mill to an average particle diameter of 1.0 µm to obtain Dispersion A-1.

In a similar way, the following color developer dispersions were made as comparison:

| Dispersion | Compound |
|---|---|
| A-2 | 2,2-bis(4-hydroxyphenyl)propane (Bisphenol A) |
| A-3 | 4,4'-dihydroxydiphenyl sulfone (Bisphenol S) |
| A-4 | 4-isopropoxy-4'-hydroxydiphenyl-sulfone (D8) |

### Preparation of color developer dispersion A-5

A mixture of 2.5 g 5-(N-3-methylphenyl-sulfonylamido)-(N',N"-bis-(3-methylphenyl)-isophthalic acid-diamide (synthesized as described in example 4), 1.7 g Gohsenx™ L-3266 (sulfonated polyvinyl alcohol, Nippon Gohsei) as dispersing aid and binder and 6.8 g demineralized water was milled in a bead mill to an average particle diameter of 1.0 µm to obtain dispersion A-5.

In a similar way the following color developer dispersions were made:

| Dispersion | Compound from example |
|---|---|
| A-6 | 2 |
| A-7 | 6 |
| A-8 | 7 |
| A-9 | 8 |
| A-10 | 9 |
| A-11 | 10 |
| A-12 | 11 |
| A-13 | 12 |
| A-14 | 13 |
| A-15 | 14 |
| A-16 | 15 |
| A-17 | 16 |
| A-18 | 17 |
| A-19 | 18 |
| A-20 | 19 |
| A-21 | 20 |
| A-22 | 21 |
| A-23 | 22 |
| A-24 | 23 |
| A-25 | 24 |
| A-26 | 25 |

### Preparation of color former dispersion B-1

A mixture of 5 g of 3-dibutylamino-6-methyl-7-anilnofluoran (Pergascript Black 2C, BASF SE), 10 g of a 10% by weight solution of a polyvinyl alcohol (Mowiol® 40-88, polyvinylalcohol, Mw∼ 205.000 g/mol, Sigma-Aldrich Inc. / Kuraray Europe GmbH), 0.1 g of the surfactant 2,4,7,9-tetramethyl-5-decyne-4,7-diol (Surfynol® 131 from Air Products) as 20% solution in isopropanol and 4.9 g of water was milled in a bead mill to an average particle diameter of 1.0 µm to obtain Dispersion B-1.

In a similar way the following color former dispersions were made:

| Dispersion | Compound |
|---|---|
| B-2 | 3-(N-ethyl-N-isoamylamino)-6-methyl-7-anilinofluoran (S205) |
| B-3 | 3-(N-ethyl-N-p-tolylamino)-6-methyl-7-anilinofluoran (ETAC) |

### Preparation of sensitizer dispersion C-1

A mixture of 5 g of benzyl-2-naphthyl ether (Pergaspeed 305, BASF SE), 0.1 g of a 45% solution of dispersing agent (sodium salt of naphthalene sulfonic acid condensation product with formaldehyde, TAMOL® NN 9401 from BASF SE) in water, 1.7 g of a 10% by weight solution of a polyvinyl alcohol (Mowiol® 40-88 (Polyvinylalcohol, Mw∼ 205.000, Sigma-Aldrich Inc. / Kuraray Europe GmbH) and 13.2 g of water is milled in a bead mill to an average particle diameter of 1.0 µm to obtain Dispersion C-1.

In a similar way the following color former dispersions were made:

| Dispersion | Compound |
|---|---|
| C-2 | Stearamide (> 90%, TCl) |
| C-3 | Diphenylsulfone (99+%, Alfa Aesar) |
| C-4 | 1,2-Diphenoxyethane (99%, Sigma-Aldrich Inc.) |
| C-5 | Ethyleneglycol-m-tolylether (KS-232, Sanko Chemical) |

### Preparation of filler dispersion D-1

A mixture of 10 g precipitated calcium carbonate (>99%, pro analysi, E. Merck Darmstadt), 0.1 g of an aqueous solution of a dispersing agent (sodium polyacrylate (DISPEX® AA 4140 from BASF SE), pH 7.5, active content 40% by weight), and 59.6 g of water was milled in a bead mill to an average particle diameter of 1.0 µm to obtain Dispersion D-1.

In a similar way the following filler dispersions were made:

| Dispersion | Compound |
|---|---|
| D-2 | kaoline (Ph. Eur., ∼46% SiO₂, ∼39% Al₂O₃, Fluka) |
| D-3 | Aluminium trihydroxide (Martinal OL-107 LEO, Martinswerk GmbH, Germany) |
| D-4 | amorphous silica (Sipernat ® 350, EVONIK Resource Efficiency GmbH, Germany), solid content reduced to 8.75 g |

### Application examples:

### Application example 1: preparation of a heat-sensitive recording layer coating compositions

80 g of dispersion A-1, 40 g of dispersion B-1, 80 g of dispersion C-1, 63 g of dispersion D-1, 130 g of a 10% by weight aqueous solution of a polyvinyl alcohol (Mowiol® 40-88, polyvinylalcohol, Mw∼ 205.000 g/mol, Sigma-Aldrich Inc. / Kuraray Europe GmbH) and 32.3 g of a 17% aqueous dispersion of zinc stearate (Hidorin® F115 from Chukyo Europe) were mixed and stirred to obtain a heat sensitive recording layer coating composition.
A base paper coated with calcined kaolin (Ansilex® 93 from BASF SE, coatweight 7 g/m²) was coated with the above heat sensitive recording layer coating composition using a #2 wire bar (12 µm wet thickness) and dried at room temperature. The resulting heat sensitive recording layer coating composition was calendered to obtain a smooth surface. Application example 1 was repeated with different components to yield additional heat sensitive recording layer coating compositions. Table A summarizes the different compositions used.

In a similar way, the following heat sensitive coated papers were made by exchanging the color developer dispersion A-1 by the color developer dispersion mentioned in the table below:

| Application example | Color developer |
|---|---|
| AEx-2 | A-2 |
| AEx-3 | A-3 |
| AEx-4 | A-4 |
| AEx-5 | A-5 |
| AEx-6 | A-6 |
| AEx-7 | A-7 |
| AEx-8 | A-8 |
| AEx-9 | A-9 |
| AEx-10 | A-10 |
| AEx-11 | A-11 |
| AEx-12 | A-12 |
| AEx-13 | A-13 |
| AEx-14 | A-14 |
| AEx-15 | A-15 |
| AEx-16 | A-16 |
| AEx-17 | A-17 |
| AEx-18 | A-18 |
| AEx-19 | A-19 |
| AEx-20 | A-20 |
| AEx-21 | A-21 |
| AEx-22 | A-22 |
| AEx-23 | A-23 |
| AEx-24 | A-24 |
| AEx-25 | A-25 |
| AEx-26 | A-26 |

### Application example 27:

80 g of dispersion A-11, 40 g of dispersion B-1, 80 g of dispersion C-5, 63 g of dispersion D-1, 130 g of a 10% by weight aqueous solution of a polyvinyl alcohol (Mowiol® 40-88, polyvinylalcohol, Mw∼ 205.000 g/mol, Sigma-Aldrich Inc / Kuraray Europe GmbH) and 32.3 g of a 17% aqueous dispersion of zinc stearate (Hidorin® F115 from Chukyo Europe) were mixed and stirred to obtain a heat sensitive recording layer coating composition.
A base paper coated with calcined kaolin (Ansilex® 93 from BASF SE, coatweight 7 g/m²) was coated with the above heat sensitive recording layer coating composition using a #2 wire bar (12 µm wet thickness) and dried at room temperature. The resulting heat sensitive recording layer coating composition was calendered to obtain a smooth surface. Application example 27 was repeated with different components to yield additional heat sensitive recording layer coating compositions. Table A summarizes the different compositions used.

| Application example | Color developer | Sensitizer |
|---|---|---|
| AEx-28 | A-5 | C-5 |
| AEx-29 | A-6 | C-5 |
| AEx-30 | A-18 | C-5 |
| AEx-31 | A-19 | C-5 |
| AEx-32 | A-13 | C-5 |
| AEx-33 | A-2 | C-5 |
| AEx-34 | A-24 | C-5 |
| AEx-35 | A-5 | C-2 |
| AEx-36 | A-5 | C-3 |

### Application examples 37 - 49:

20 g of color developer dispersion, 10 g of color former dispersion, 20 g of sensitizer dispersion, 30 g of filler dispersion were mixed with 14.5 g of a 10% by weight aqueous solution of a polyvinyl alcohol (Mowiol® 40-88, polyvinylalcohol, Mw∼ 205.000 g/mol, Sigma-Aldrich Inc. / Kuraray Europe GmbH) and 5.5 g of a 17% aqueous dispersion of zinc stearate (Hidorin® F115 from Chukyo Europe) and stirred to obtain a heat sensitive recording layer coating composition. The nature and amounts of the dispersions used are summarised in table A.
A base paper coated with calcined kaolin (Ansilex® 93 from BASF SE, coatweight 7 g/m²) was coated with the above heat sensitive recording layer coating composition using a #2 wire bar (12 µm wet thickness). The coated paper was subsequently dried at room temperature. The resulting heat sensitive recording layer coating composition was calendered to obtain a smooth surface.

| Application example | Color former | | Color developer | | Sensitizer | | Filler | | OBA |
|---|---|---|---|---|---|---|---|---|---|
| | Nr. | amount | Nr. | amount | Nr. | amount | Nr. | amount | amount |
| AEx-37 | B-1 | 10 | A-5 | 20 | C-1 | 20 | D-1 | 30 | - |
| AEx-38 | B-1 | 10 | A-5 | 20 | C-1 | 20 | D-2 | 30 | - |
| AEx-39 | B-1 | 10 | A-5 | 20 | C-1 | 20 | D-3 | 30 | - |
| AEx-40 | B-1 | 10 | A-5 | 20 | C-1 | 20 | D4 | 30 | - |
| AEx-41 | B-2 | 10 | A-5 | 20 | C-1 | 20 | D-1 | 30 | |
| AEx-42 | B-1 | 5 | A-5 | 20 | C-1 | 20 | D-1 | 30 | - |
| | B-2 | 5 | | | | | | | |
| AEx-43 | B-1 | 6 | A-5 | 20 | C-1 | 20 | D-1 | 30 | - |
| | B-2 | 4 | | | | | | | |
| AEx-44 | B-1 | 10 | A-5 | 20 | C-2 | 20 | D-1 | 30 | - |
| AEx-45 | B-1 | 10 | A-5 | 20 | C-3 | 20 | D-1 | 30 | |
| AEx-46 | B-1 | 10 | A-5 | 20 | C-4 | 20 | D-1 | 30 | - |
| AEx-47 | B-3 | 10 | A-5 | 30 | C-1 | 20 | D-1 | 30 | - |
| AEx-48 | B-3 | 10 | A-5 | 30 | C-1 | 20 | D-3 | 30 | - |
| AEx-49 | B-1 | 10 | A-5 | 20 | C-1 | 20 | D-1 | 30 | 0.3 * |

| | | | | | | | | | |
|---|---|---|---|---|---|---|---|---|---|
| *Tinopal® ABP-X HC new (BASF India Ltd.) was used as optical brightening agent and added as is to the final heat sensitive coating composition. | | | | | | | | | |

Amounts mentioned in the table are parts by weight.

### Evaluation of Heat Sensitive Recording Materials

The heat sensitive recording materials prepared according to the invention were evaluated as described below and the results of the evaluations are summarized in Table A.

### Image Optical Density

Using a Thermal Tester (Atlantek Model 400 manufactured by Atlantek Inc.), each heat sensitive recording material was printed at an applied energy of 30 mJ/mm² and the density of the recorded image thus obtained was measured with a GretagMacbeth™ eyeone pro densitometer.

### Background

The optical density of the unrecorded portion of the heat sensitive material was measured with a densitometer (GretagMacbeth™ eyeone pro densitometer).

### Heat Resistance

After printing, the heat sensitive recording material was stored for 24 hours in an oven at a temperature of 60°C. The optical densities of the recorded and unrecorded portions were then measured with the above densitometer.

### Heat / Humidity Resistance

After printing, the heat sensitive recording material was stored for 24 hours in an oven at a temperature of 40°C and 90% relative humidity. The optical densities of the recorded and unrecorded portions were then measured with the above densitometer.

### Light Resistance

After printing, the heat sensitive recording material was stored for 4.5 hours in a xenon weatherometer (Atlas Suntester GPS+, 1000 W/m²). The optical densities of the recorded and unrecorded portions were then measured with the above densitometer.

### Oil Resistance

After printing, the heat sensitive recording material was coated using a #2 wire bar (12 µm wet thickness) with cottonseed oil and then stored for 24 hours in an oven at a temperature of 40°C. The optical density of the recorded portion was then measured with the above densitometer.

**Table A - Part 1: Evaluation of heat sensitive recording material using benzyl-2-naphthyl ether as sensitizer**

| Appl. example | | Optical density (recorded portion) | | Optical density (unrecorded portion) | | Heat resistance (recorded portion) | | Heat resistance (unrecorded portion) | |
|---|---|---|---|---|---|---|---|---|---|
| AEx-1 | | 1.41 | | 0.05 | | 1.35 (96%) | | 0.1 | |
| AEx-2 | | 1.28 | | 0.05 | | 1.11 (87%) | | 0.13 | |
| AEx-3 | | 1.51 | | 0.07 | | 1.32 (87%) | | 0.09 | |
| AEx-4 | | 1.52 | | 0.06 | | 1.39 (91%) | | 0.12 | |
| AEx-5 | | 0.91 | | 0.03 | | 0.53 (58%) | | 0.06 | |
| AEx-6 | | 1.37 | | 0.08 | | 1.20 (88%) | | 0.11 | |
| AEx-7 | | 1.43 | | 0.13 | | 1.32 (92%) | | 0.4 | |
| AEx-11 | | 1.16 | | 0.04 | | 1.24 (107%) | | 0.11 | |
| AEx-13 | | 1.20 | | 0.05 | | 1.18 (98%) | | 0.07 | |
| AEx-14 | | 0.86 | | 0.05 | | 0.19 (22%) | | 0.07 | |
| AEx-18 | | 1.24 | | 0.09 | | 1.20 (97%) | | 0.1 | |
| AEx-19 | | 1.49 | | 0.11 | | 1.48 (99%) | | 1.04 | |
| AEx-20 | | 1.41 | | 0.1 | | 1.51 (93%) | | 0.58 | |
| AEx-21 | | 1.37 | | 0.15 | | 1.42 (104%) | | 0.78 | |
| AEx-22 | | 1.31 | | 0.05 | | 0.58 (44%) | | 0.06 | |
| | | | | | | | | | |

| Appl. example | Heat / humidity resistance (recorded portion) | | Heat / humidity resistance (unrecorded portion) | | Light resistance (recorded portion) | | Light resistance (unrecorded portion) | | Oil resistance (recorded portion) |
|---|---|---|---|---|---|---|---|---|---|
| AEx-1 | 1.26 (89%) | | 0.13 | | 1.33 (94%) | | 0.15 | | 1.47 (104%) |
| AEx-2 | 1.23 (96%) | | 0.08 | | 1.11 (87%) | | 0.10 | | 0.98 (77%) |
| AEx-3 | 1.19 (79%) | | 0.11 | | 1.58 (105%) | | 0.18 | | 1.03 (68%) |
| AEx-4 | 1.39 (91%) | | 0.08 | | 1.54 (101%) | | 0.15 | | 0.31 (20%) |
| AEx-5 | - | | - | | 1.06 (116%) | | 0.11 | | - |
| AEx-6 | 1.27 (93%) | | 0.09 | | 1.35 (98%) | | 0.15 | | 1.22 (89%) |
| AEx-7 | 1.48 (103%) | | 0.33 | | 1.52 (106%) | | 0.45 | | - |
| AEx-11 | - | | - | | 1.28 (110%) | | 0.13 | | 1.15 (99%) |
| AEx-13 | 0.80 (67%) | | 0.05 | | 1.18 (98%) | | 0.18 | | 1.04 (87%) |
| AEx-14 | 0.13 (15%) | | 0.07 | | 0.95 (110%) | | 0.15 | | 0.14 (16%) |
| AEx-18 | 1.30 (105%) | | 0.11 | | 1.37 (110%) | | 0.18 | | 0.97 (78%) |
| AEx-19 | 1.43 (96%) | | 0.45 | | 1.49 (100%) | | 0.16 | | 0.73 (49%) |
| AEx-20 | 1.36 (96%) | | 0.33 | | 1.43 (101%) | | 0.29 | | - |
| AEx-21 | 1.41 (103%) | | 0.54 | | 1.43 (104%) | | 0.29 | | - |
| AEx-22 | - | | - | | 1.41 (108%) | | 0.13 | | - |

**Table A - Part 2: Evaluation of heat sensitive recording material using ethylene glycol-bis-m-tolylether, stearylamide or diphenylsulfone as sensitizer**

| Appl. example | Optical density (recorded portion) | Optical density (unrecorded portion) | Heat resistance (recorded portion) | Heat resistance (unrecorded portion) | |
|---|---|---|---|---|---|
| AEx-27 | 1.2 | 0.04 | 0.3 (25%) | 0.13 | |
| AEx-28 | 0.86 | 0.04 | 0.32 (37%) | 0.05 | |
| AEx-29 | 1.22 | 0.04 | 0.92 (75%) | 0.06 | |
| AEx-30 | 1.24 | 0.09 | 0.99 (80%) | 0.12 | |
| AEx-31 | 1.42 | 0.13 | 1.43 (101%) | 0.89 | |
| AEx-32 | 1.03 | 0.05 | 0.99 (96%) | 0.06 | |
| AEx-33 | 1.52 | 0.10 | 1.15 (76%) | 0.27 | |
| AEx-34 | 0.80 | 0.04 | 1.17 (146%) | 0.11 | |
| AEx-35 | 0.80 | 0.03 | 0.60 (75%) | 0.05 | |
| AEx-36 | 0.88 | 0.04 | 0.46 (52%) | 0.05 | |

| Appl. example | Heat / humidity resistance (recorded portion) | Heat / humidity resistance (unrecorded portion) | Light resistance (recorded portion) | Light resistance (unrecorded portion) | Oil resistance (recorded portion) |
|---|---|---|---|---|---|
| AEx-27 | 0.88 (73%) | 0.08 | 1.31 (109%) | 0.13 | 1.10 (92%) |
| AEx-28 | - | - | 1.05 (122%) | 0.13 | 0.15 (17%) |
| AEx-29 | 0.91 (74%) | 0.05 | 1.24 (102%) | 0.11 | 0.28 (23%) |
| AEx-30 | 1.15 (93%) | 0.09 | 1.24 (100%) | 0.17 | 0.19 (15%) |
| AEx-31 | 1.37 (96%) | 0.32 | 1.47 (103%) | 0.13 | 1.46 (103%) |
| AEx-32 | 0.81 (79%) | 0.05 | 1.18 (115%) | 0.16 | 0.80 (78%) |
| AEx-33 | 1.15 (76%) | 0.16 | 1.50 (99%) | 0.17 | 0.26 (17%) |
| AEx-34 | 1.09 (136%) | 0.09 | 1.25 (156%) | 0.18 | - |
| AEx-35 | 0.53 (66%) | 0.04 | 0.89 (111%) | 0.13 | 0.13 (16%) |
| AEx-36 | 0.41 (46%) | 0.04 | 1.04 (118%) | 0.13 | 0.11 (12%) |

Investigation of the influence of different coating components on the performance of 5-(N-3-methylphenyl-sulfonylamido)-(N',N"-bis-(3-methylphenyl)-isophthalic acid-diamide as sensitizer as made in example 4.

| Appl. example | Optical density (recorded portion) | Optical density (unrecorded portion) | Heat resistance (recorded portion) | Heat resistance (unrecorded portion) | |
|---|---|---|---|---|---|
| AEx-37 | 1.33 | 0.10 | 1.20 (90%) | 0.14 | |
| AEx-38 | 1.32 | 0.11 | 1.14 (86%) | 0.13 | |
| AEx-39 | 1.35 | 0.10 | 1.20 (89%) | 0.14 | |
| AEx-40 | 1.29 | 0.10 | 1.12 (87%) | 0.16 | |
| AEx-41 | 1.33 | 0.10 | 1.25 (94%) | 0.14 | |
| AEx-42 | 1.35 | 0.11 | 1.25 (92%) | 0.17 | |
| AEx-43 | 1.38 | 0.12 | 1.25 (91%) | 0.18 | |
| AEx-44 | 1.35 | 0.09 | 1.28 (95%) | 0.11 | |
| AEx-45 | 1.34 | 0.09 | 1.24 (93%) | 0.14 | |
| AEx-46 | 1.40 | 0.09 | 1.14 (81%) | 0.11 | |
| AEx-47 | 1.25 | 0.06 | 0.92 (74%) | 0.08 | |
| AEx-48 | 1.14 | 0.08 | 0.93 (81%) | 0.09 | |
| AEx-49 | 1.37 | 0.08 | 1.20 (88%) | 0.11 | |

| Appl. example | Heat / humidity resistance (recorded portion) | Heat / humidity resistance (unrecorded portion) | Light resistance (recorded portion) | Light resistance (unrecorded portion) | Oil resistance (recorded portion) |
|---|---|---|---|---|---|
| AEx-37 | 1.28 | 0.11 | 1.39 | 0.09 | 1.22 |
| AEx-38 | 1.28 | 0.10 | 1.33 | 0.11 | 1.32 |
| AEx-39 | 1.29 | 0.10 | 1.33 | 0.11 | 1.44 |
| AEx-40 | 1.19 | 0.10 | 1.27 | 0.10 | 0.52 |
| AEx-41 | 1.26 | 0.12 | 1.35 | 0.12 | 1.12 |
| AEx-42 | 1.30 | 0.13 | 1.35 | 0.14 | 1.44 |
| AEx-43 | 1.31 | 0.14 | 1.39 | 0.14 | 1.00 |
| AEx-44 | 1.28 | 0.11 | 1.31 | 0.16 | 0.71 |
| AEx-45 | 1.27 | 0.10 | 1.36 | 0.15 | 1.41 |
| AEx-46 | 1.31 | 0.08 | 1.37 | 0.13 | 1.24 |
| AEx-47 | 0.99 | 0.06 | 1.21 | 0.11 | 0.43 |
| AEx-48 | 0.94 | 0.06 | 1.19 | 0.12 | 0.41 |
| AEx-49 | 1.27 | 0.09 | 1.35 | 0.08 | 1.22 |

## Claims

1. A heat sensitive recording material, comprising
a) at least a color forming compound, and
b) at least a color developer of the formula (I) 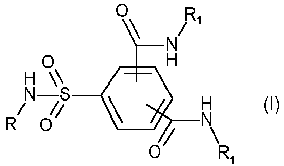 wherein
R and R₁ are each independently of each other hydrogen, C₁-C₁₈-alkyl, C₁-C₈-alkoxy-C₁-C₈-alkyl, (R₉)₂N-C₁-C₈-alkyl, wherein Rg stands for C₁-C₈-alkyl or C₅-C₆-cycloalkyl, or a radical of formula (II) 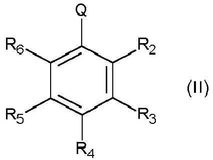 wherein R₂, R₃, R₄, R₅, R₆ are each independently of each other hydrogen, C₁-C₈-alkyl, -NH-C(=O)-R₇, -C(=O)-NH-R₇, wherein R₇ stands for C₁-C₈-alkyl, -C(=O)OR₈, wherein R₈ stands for C₁-C₈-alkyl, halogen, or wherein R₂ and R₃, or R₄ and R₅ or both, or wherein R₃ and R₄, or R₅ and R₆ or both, or wherein (R₂ and R₃) as well as (R₅ and R₆), can stand together for a hydrocarbon diradical with 3 or 4 carbon atoms,
and Q stands for a single bond or C₁-C₈-alkylene, which can be branched or unbranched and wherein the main chain may contain one or more oxygen atoms between two carbon atoms in case of more than 2 carbon atoms.

2. Compound of formula (I).

3. Compound of formula (Ia)

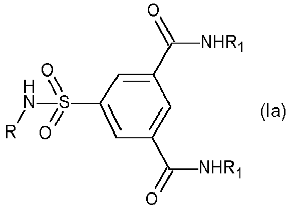

4. Process for the manufacture of a compound with formula (I), wherein R and R₁ are the same, comprising reacting an acid chloride of formula (IVb) 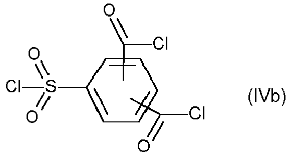 with an amine RNH₂.

5. The process according to claim 4, wherein the acid chloride (IVb) is 5-sulfonyl chloride-isophthalic acid dichloride.

6. Process for the manufacture of a compound with formula (I), wherein R and R₁ are different, comprising the steps of preparing a compound of formula (V), 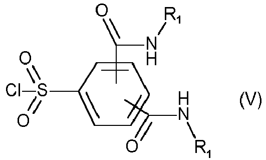 the preparation comprising
a) reacting an acid chloride of formula (IVb) with a first amine R₁NH₂,
b) reacting the obtained compound (V) with a second amine RNH₂.

7. The process according to claim 6, wherein the acid chloride (IVb) is 5-sulfonyl chloride-isophthalic acid dichloride.

8. Process for the manufacture of compound (I) comprising the steps of
a) preparing an acid chloride of formula (IVb), the preparation comprising reacting a sulfo-isophthalic compound (IVa) 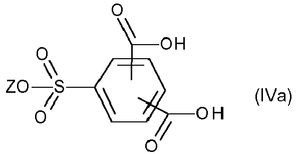 wherein Z stands for stands for hydrogen or an alkali metal, with a chlorination agent,
b) optionally isolating the obtained acid chloride (IVb),
c) reacting the acid chloride (IVb) with an amine RNH2.

9. Use of compound (I) as color developer in a heat sensitive recording material.

10. A heat sensitive recording material according to claim 1 further comprising at least one sensitizer.

11. A heat sensitive recording material according to claims 1 and 10 further comprising at least one stabilizer.
